# EUROPEAN PATENT APPLICATION

(11) **EP 3 075 381 A1**
(43) Date of publication of application: **05.10.2016**
(21) Application number: 15305508.2
(22) Date of filing: 03.04.2015
(51) Int. Cl.: A61K 31/439, A61P 31/04, C07D 471/18, C07D 513/18

(54) **HETEROCYCLIC COMPOUNDS AND THEIR USE IN PREVENTING OR TREATING BACTERIAL INFECTIONS**

(71) Applicant: Mutabilis, 75007 Paris (FR)
(72) Inventor: Brias, Julie, 75005 PARIS (FR); Chasset, Sophie, 77176 NANDY (FR); Chevreuil, Francis, 60500 CHANTILLY (FR); Lecointe, Nicolas, 75018 PARIS (FR); Ledoussal, Benoît, 22450 POMMERIT JAUDY (FR); Le Strat, Frédéric, 77380 COMBS LA VILLE (FR)
(74) Representative: Lavoix

(57) **Abstract**

The present invention relates to heterocyclic compounds of formula (I), their process of preparation, pharmaceutical compositions comprising these compounds and use thereof, optionally in combination with other antibacterial agents and/or beta-lactam compounds, for the prevention or treatment of bacterial infections. The present invention also relates to the use of these compounds as β-lactamase inhibitors and/or as antibacterial agents.

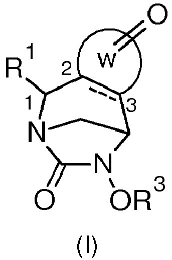

## Description

The present invention relates to heterocyclic compounds, their process of preparation, pharmaceutical compositions comprising these compounds and use thereof, optionally in combination with other antibacterial agents and/or beta-lactam compounds, for the prevention or treatment of bacterial infections. The present invention also relates to the use of these compounds as β-lactamase inhibitors and/or as antibacterial agents.

It has been described that there is a continuous evolution of antibacterial resistance which could lead to bacterial strains against which known antibacterial compounds are inefficient.

There is thus a need to provide effective compounds and composition that can overcome bacterial antibiotic resistance.

The objective of the present invention is to provide heterocyclic compounds that can be used as antibacterial agents and/or beta-lactamase inhibitors.

An objective of the present invention is also to provide heterocyclic compounds that can be used for the prevention or for the treatment of bacterial infections.

Another objective of the present invention is to provide heterocyclic compounds that can overcome bacterial antibiotic resistance.

An objective of the invention is also to provide pharmaceutical compositions comprising such heterocyclic compounds, optionally in combination with one or more other antibacterial agent, for the prevention or for the treatment of bacterial infections and which can overcome bacterial antibiotic resistance.

Other objectives will appear throughout the description of the invention.

The present invention thus provides a compound of formula (I) 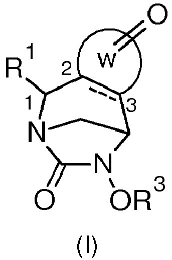 wherein
- W, unsubstituted or substituted by one or more T, represents a non-aromatic, unsaturated 5- or 6-membered heterocycle comprising at least one group N-R² and a group (X)ₙ ;
- X, identical or different, independently represents C(O), O, N, N(R²), S, S(O) or S(O)₂ ;
- R¹ represents a carbon-linked, unsubstituted or substituted by one or more T¹, aromatic, saturated, totally or partially unsaturated 4-, 5- or 6-membered heterocycle comprising at least one nitrogen atom ; a hydrogen atom ; -CN ; - C(O)NHQ¹ ; -C(O)NHOQ¹ ; -C(O)NH-NHQ¹ ; -C(O)O-NHQ¹ ; -C(O)OQ¹ ; - (CH₂)ₘOC(O)OQ¹ ; -(CH₂)ₘOQ¹ ; -(CH₂)ₘOC(O)Q¹ ; -(CH₂)ₘOC(O)NQ¹Q² ; - (CH₂)ₘ-NHC(O)Q¹ ; -(CH₂)ₘNHS(O)₂Q¹ ; -(CH₂)ₘNHS(O)₂NQ¹Q² ; - (CH₂)ₘNHC(O)OQ¹ ; -(CH₂)ₘNHC(O)NQ¹Q² ; -(CH₂)ₘNHQ³ ; -(CH₂)ₘNH-C(NHQ³)=NQ⁴ ; -(CH₂)ₘNH-CH=NQ³ ; -C(NHQ³)=NQ⁴ ;
- R², identical or different, independently represents -(CH₂)_{q}OQ⁵ ; -(CH₂)ᵥ-CN ; - (CH₂)_{q}OC(O)Q⁵ ; -(CH₂)ᵥ-C(O)OQ⁵ ; -(CH₂)_{q}-OC(O)OQ⁵ ; -(CH₂)_{q}-OC(O)NQ⁵Q⁶ ; - (CH₂)ᵥ-C(O)NQ⁵Q⁶ ; -(CH₂)ᵥ-C(O)ONQ⁵ ; -(CH₂)ᵥ-C(O)NHOQ⁵ ; -(CH₂)ᵥ-C(O)NH-NHQ⁵ ; -(CH₂)ᵥ-C(O)O-NHQ⁵; -(CH₂)_{q}-NHC(O)Q⁵ ; -(CH₂)_{q}NHS(O)₂Q⁵ ; - (CH₂)_{q}NHS(O)₂NQ⁵Q⁶ -(CH₂)_{q}-NHC(O)OQ⁵ ; -(CH₂)_{q}-NHC(O)NQ⁵Q⁶ ; - (CH₂)_{q}NQ⁵Q⁶ ; -(CH₂)_{q}-NH-C(NHQ³)=NQ⁴ ; -(CH₂)_{q}-NH-CH=NQ³ ; (CH₂)ᵥ-C(NHQ³)=NQ⁴ ; or
   R², identical or different, independently unsubstituted or substituted by one or more T², independently represents C₁-C₃-alkyl ; C₁-C₃-fluoroalkyl ; -(CH₂)ₚ-C₃-C₆-cycloalkyl ; -(CH₂)ₚ-C₃-C₆-cyclofluoroalkyl ; -(CH₂)ₚ-(4-, 5- or 6-membered aromatic, saturated, totally or partially unsaturated heterocycle) ;
- R³ represents SO₃H, CFHCO₂H or CF₂CO₂H ;
- Q¹ and Q², identical or different, independently represent a hydrogen atom ; - (CH₂)_{q}NHQ³ ; -(CH₂)_{q}-NH-C(NHQ³)=NQ⁴ ; (CH₂)_{q}-NH-CH=NQ³ ; (CH₂)ᵥ-C(NHQ³)=NQ⁴ ; -(CH₂)_{q}OQ³ ; -(CH₂)ᵥCONHQ³ ; or
   Q¹ and Q², identical or different, independently unsubstituted or substituted by one or more T², independently represent a C₁-C₃-alkyl ; -(CH₂)ₚ-(4-, 5- or 6-membered heterocycle) ; or
   Q¹, Q² and the nitrogen atom to which they are bonded, form a saturated or partially unsaturated 4-, 5- or 6-membered heterocycle comprising 1, 2, 3 or 4 heteroatoms ;
- Q³ and Q⁴, identical or different, independently represent a hydrogen atom or a C₁-C₃-alkyl ;
- Q⁵ and Q⁶, identical or different, independently represent a hydrogen atom ; - (CH₂)_{q}NHQ³ ; -(CH₂)_{q}-NH-C(NHQ³)=NQ⁴ ; (CH₂)_{q}-NH-CH=NQ³ ; (CH₂)ᵥ-C(NHQ³)=NQ⁴ ; -(CH₂)_{q}OQ³ ; -(CH₂)ᵥCONHQ³ ; or
   Q⁵ and Q⁶, identical or different, independently unsubstituted or substituted by one or more T², independently represent a C₁-C₃-alkyl ; -(CH₂)ₚ-(4-, 5- or 6-membered heterocycle) ; or
   Q⁵, Q⁶ and the nitrogen atom to which they are bonded, form a saturated or partially unsaturated 4-, 5- or 6-membered heterocycle comprising 1, 2, 3 or 4 heteroatoms ;
- T¹, identical or different, independently represents a fluorine atom ; -(CH₂)ₚOQ¹ ; - (CH₂)ₚ-CN; -(CH₂)ₚOC(O)Q¹ ; -(CH₂)ₚ-C(O)OQ¹ ; -(CH₂)ₚ-OC(O)OQ¹ ; -(CH₂)ₚ-OC(O)NHQ¹ ; -(CH₂)ₚ-C(O)NHQ¹ ; -(CH₂)ₚ-C(O)NHOQ¹ ; -(CH₂)ₚ-C(O)NH-NHQ¹ ; - (CH₂)ₚ-C(O)O-NHQ¹ ; -(CH₂)ₚ-NHC(O)Q¹ ; -(CH₂)ₚNHS(O)₂Q¹ ; - (CH₂)ₚNHS(O)₂NQ¹Q² ; -(CH₂)ₚ-NHC(O)OQ¹ ; -(CH₂)ₚ-NHC(O)NQ¹Q² ; - (CH₂)ₚNHQ¹ ; -(CH₂)ₚ-NH-C(NHQ³)=NQ⁴ ; -(CH₂)ₚ-NH-CH=NQ³ ; (CH₂)ₚ-C(NHQ³)=NQ⁴ ; or
   T¹, unsubstituted or substituted by one or more T², identical or different, independently represents C₁-C₃-alkyl ; C₁-C₃-fluoroalkyl ; O-C₁-C₃-fluoroalkyl ; - (CH₂)ₚ-(4-, 5- or 6-membered aromatic, saturated, totally or partially unsaturated heterocycle comprising at least one nitrogen atom) ;
- T², identical or different, independently represents OH ; NH₂ or CONH₂ ;
- T, identical or different, independently represents a fluorine atom ; C₁-C₃-alkyl ; C₁-C₃-fluoroalkyl ; O-C₁-C₃-fluoroalkyl ; -(L)_{w}-(CH₂)ₚ-C₃-C₆-cycloalkyl ; -(L)_{w}-(CH₂)ₚ-C₃-C₆-cyclofluoroalkyl ; -(L)_{w}-(CH₂)ₚ-heterocycle ; -(L)_{w}-(CH₂)ₚ-CN ; -(L)_{w}-(CH₂)ₚOC(O)Q⁵; -(L)_{w}-(CH₂)ₚ-C(O)OQ⁵; -(L)_{w}-(CH₂)ₚ-OC(O)OQ⁵ ; -(L)_{w}(CH₂)ₚ-OC(O)NQ⁵Q⁶ ; -(L)_{w}-(CH₂)ₚ-C(O)NQ⁵Q⁶ ; -(L)_{w}-(CH₂)ₚ-C(O)NQ⁵OQ⁶ ; -(L)_{w}-(CH₂)ₚ-C(O)NQ⁵-NQ⁵Q⁶ ; -(L)_{w}-(CH₂)ₚ-NQ⁵C(O)Q⁶ ; -(L)_{w}-(CH₂)ₚNQ⁵Q(O)₂Q⁶ ; -(L)_{w}-(CH₂)ₚ-NQ⁵C(O)OQ⁶ ; -(L)_{w}-(CH₂)ₚ-NQ⁵C(O)NQ⁵Q⁶ ; -(L)_{w}-(CH₂)ₚNQ⁵Q⁶ ; -(L)_{w}-(CH₂)ₚ-NH-C(NHQ³)=NQ⁴ ; -(L)_{w}-(CH₂)ₚ-NH-CH=NQ³ ; -(L)_{w}(CH₂)ₚ-C(NHQ³)=NQ⁴ ;
- L, identical or different, independently represents O, S, N(R²), S(O) or S(O)₂ ;
- m represents 1 or 2 ;
- n represents 0, 1 or 2 ;
- p, identical or different, independently represents 0, 1, 2 or 3 ;
- q, identical or different, independently represents 2 or 3 ;
- v, identical or different, independently represents 1, 2 or 3 ;
- w, identical or different, independently represents 0 or 1 ;
wherein
▪ any carbon atom present within a group selected from alkyl, cycloalkyl, fluoroalkyl, cyclofluoroalkyl and heterocycle can be oxidized to form a C=O group ;
▪ any sulphur atom present within a heterocycle can be oxidized to form a S=O group or a S(O)₂ group ;
▪ any nitrogen atom present within a heterocycle or present within group wherein it is trisubstituted thus forming a tertiary amino group, can be further quaternized by a methyl group ;
and a pharmaceutically acceptable salt, a zwitterion, an optical isomer, a racemate, a diastereoisomer, an enantiomer, a geometric isomer or a tautomer thereof.

For the compounds according to the invention, W, unsubstituted or substituted by one or more T, represents a non-aromatic, unsaturated 5- or 6-membered heterocycle comprising at least one group N-R² and a group (X)ₙ. According to the invention, heterocycle W comprises a double bond between the 2- and 3-position carbon atoms or two double bonds on these 2- and 3-position carbon atoms. The carbon atoms in the 2- and 3-position are thus different from sp³ carbon atoms.

According to the invention, heterocycle W comprises at least one heteroatom that is a nitrogen atom. Heterocycle W can further comprise one or two further heteroatoms. These further heteroatoms can be selected from an oxygen atom, a nitrogen atom and a sulfur atom. The carbon atoms or the sulfur atoms possibly present in heterocycle W may be oxidized to form C(O) groups, S(O) or S(O)₂ groups.

According to the invention, heterocycle W is a monocycle.

Preferably, the compound according to the invention is selected from the compounds of formulae (A) and (B) 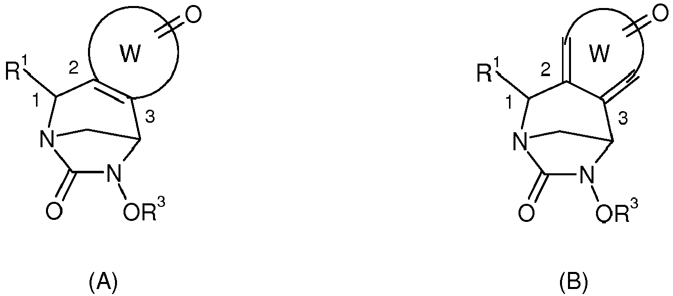 wherein W, unsubstituted or substituted by one or more T, X, n, R¹, R² and R³ are defined according to formula (I).

Also preferably, the compound according to the invention is selected from the compounds of formulae (A1) to (A68) and (B1) to (B8) 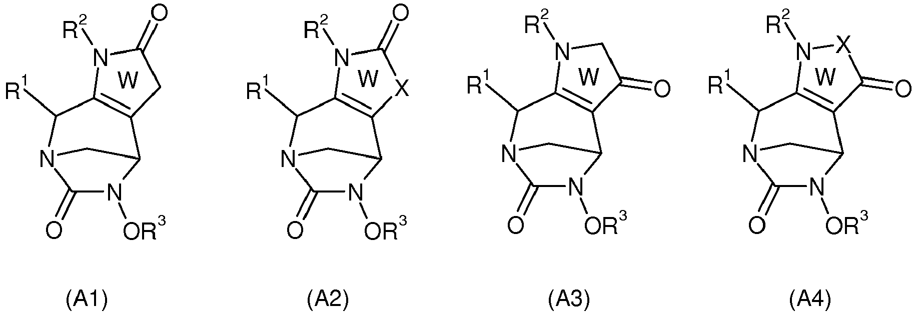 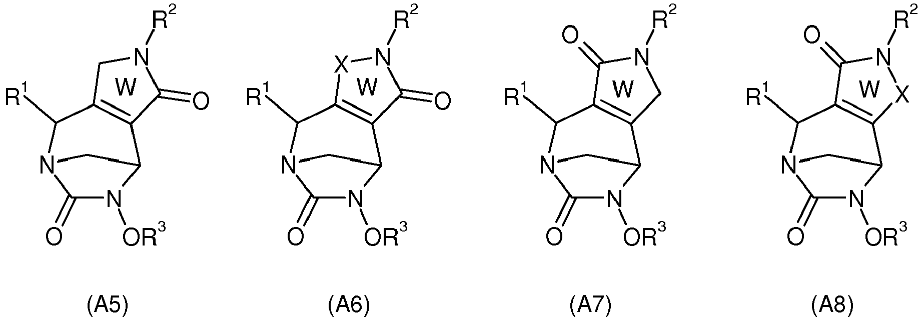 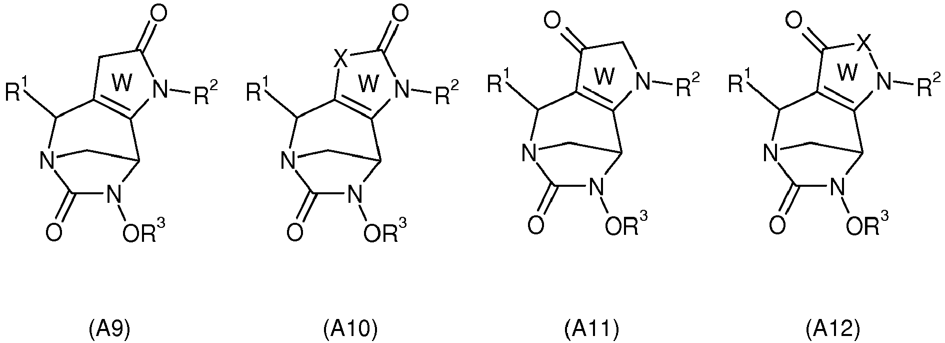 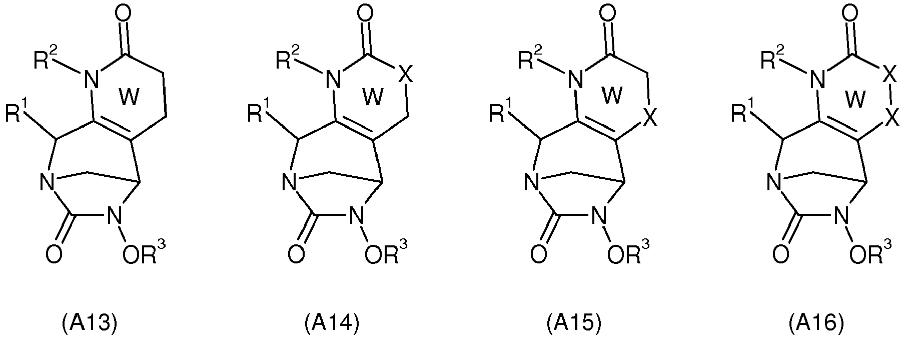 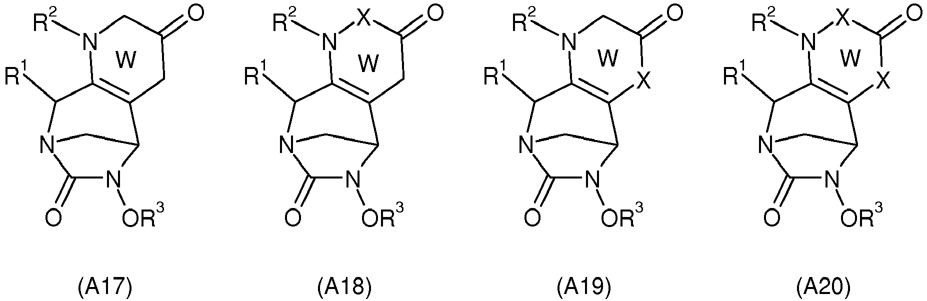 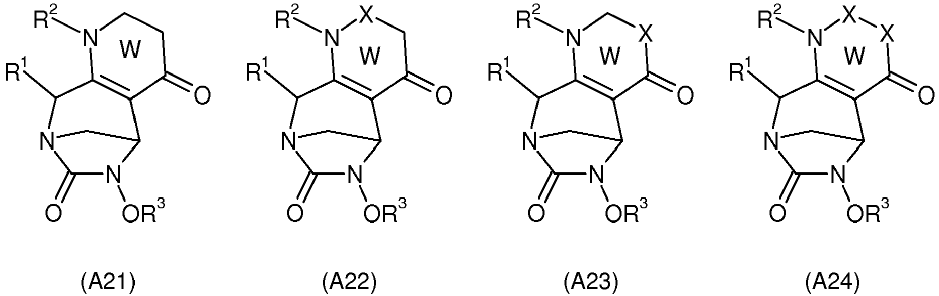 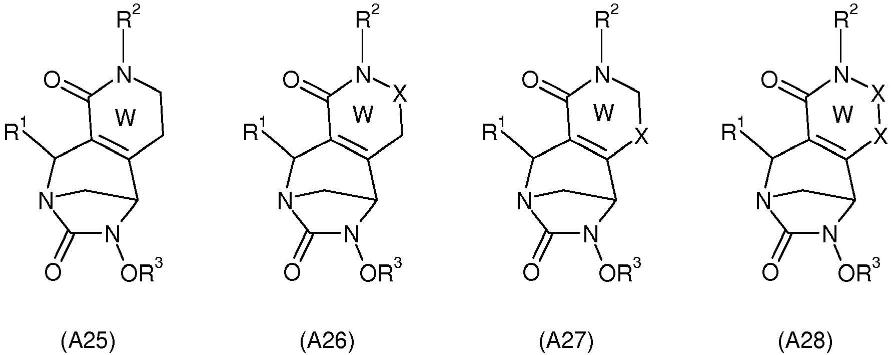 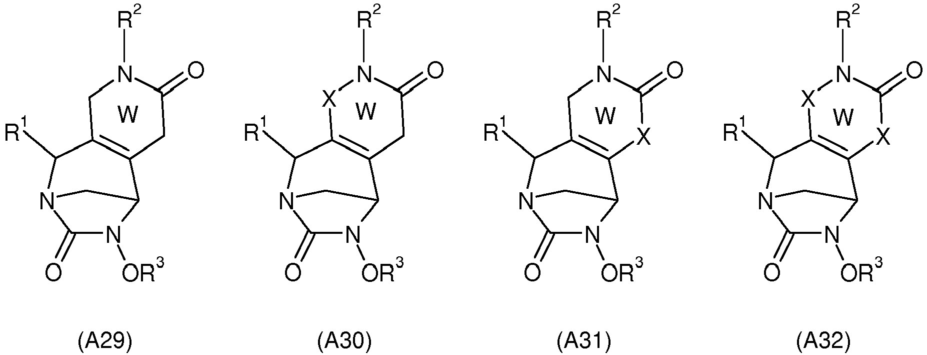 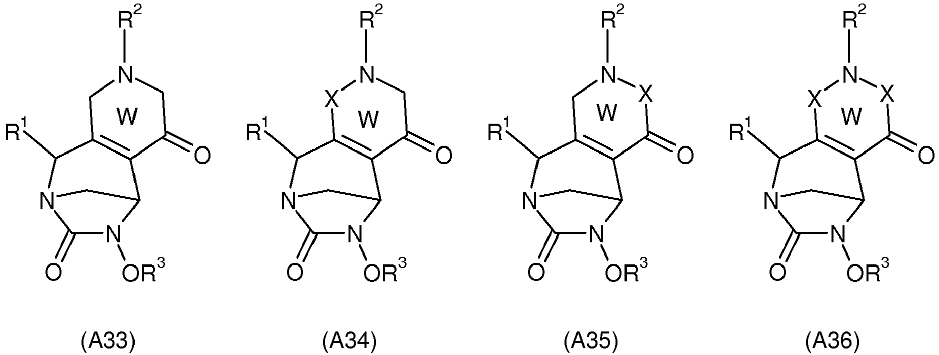 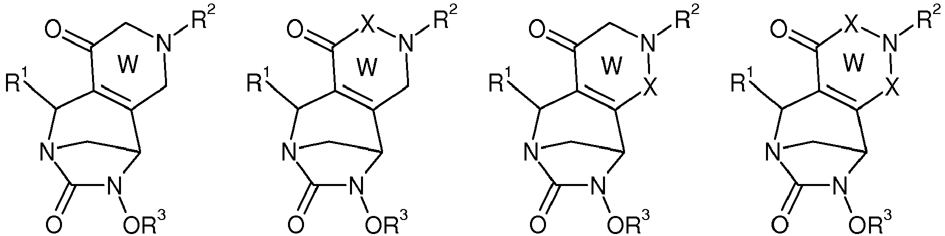 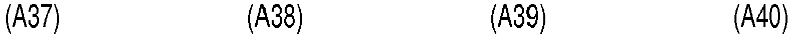 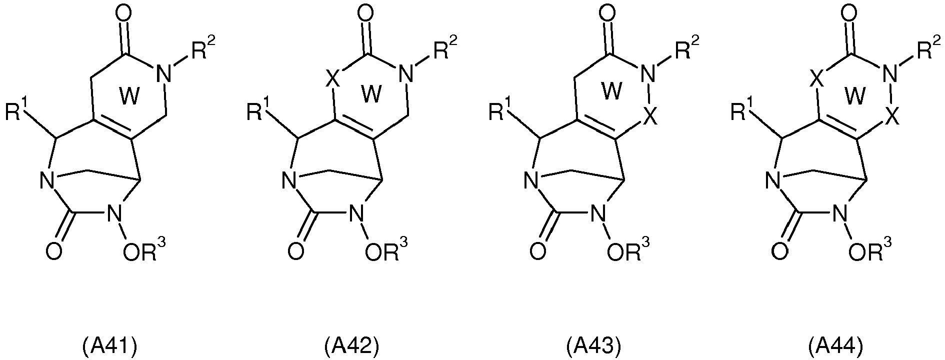 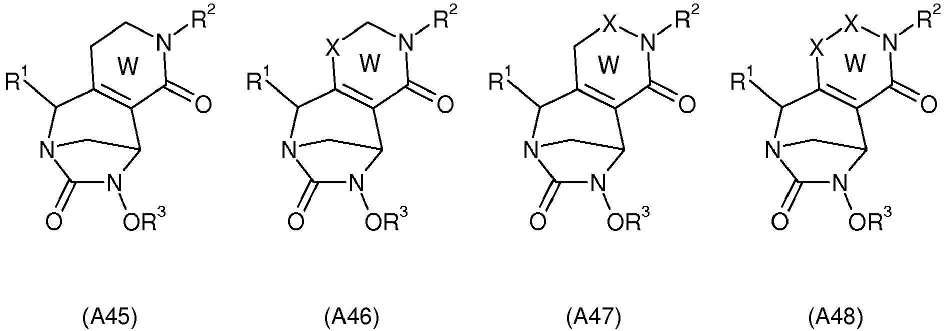 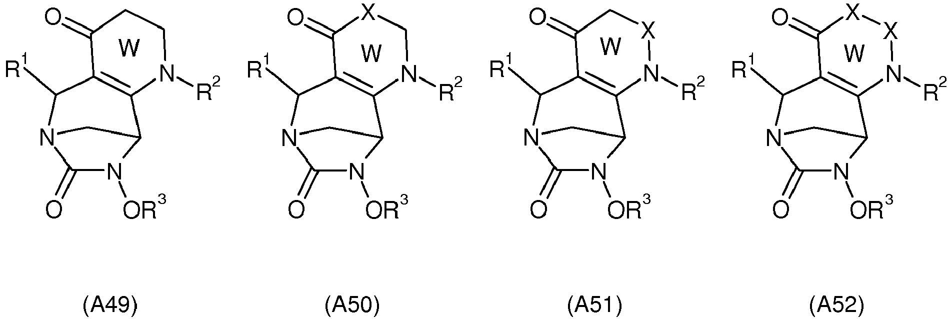 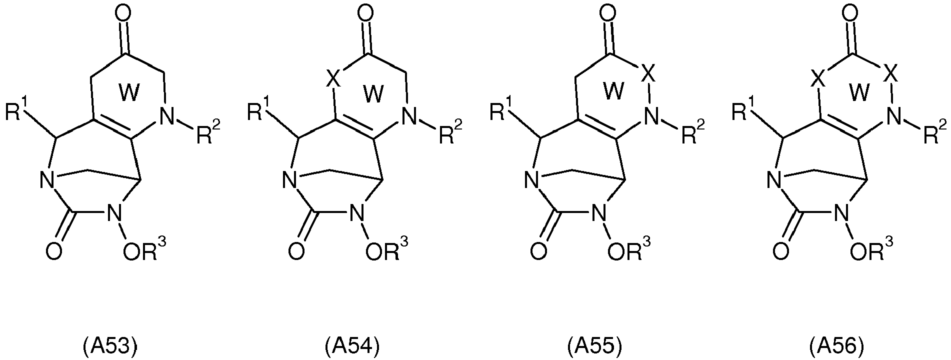 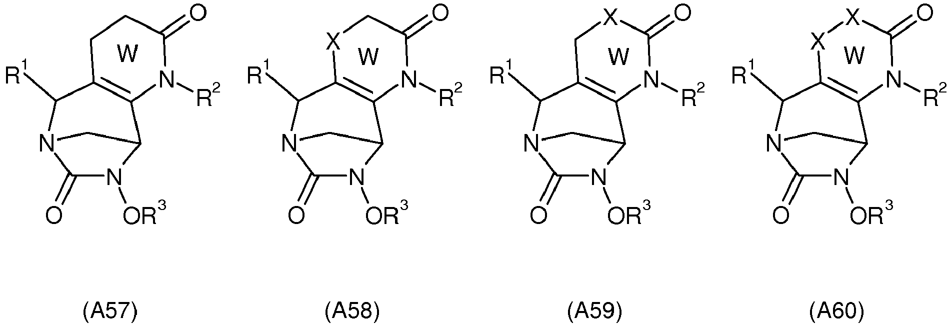 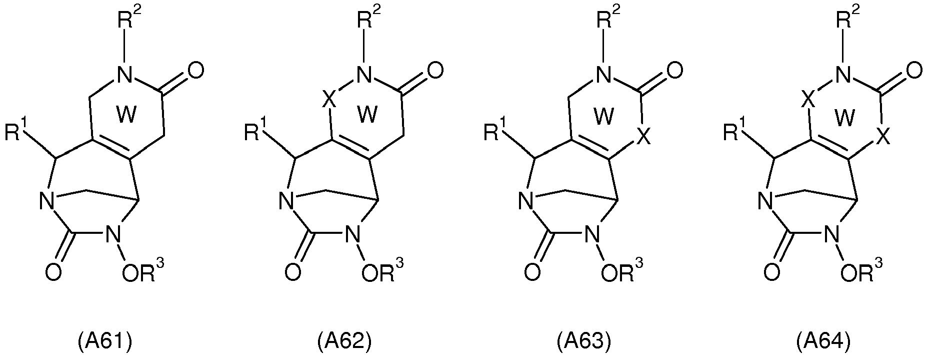 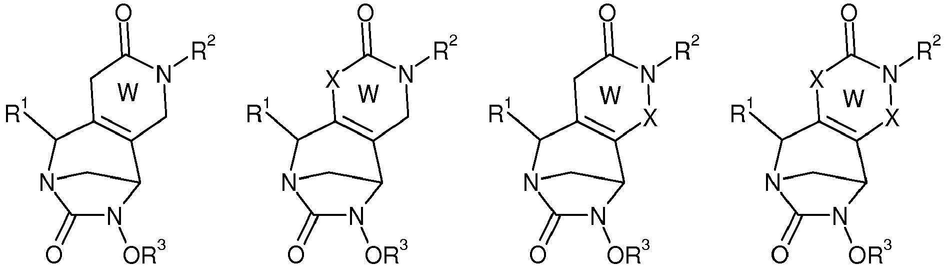 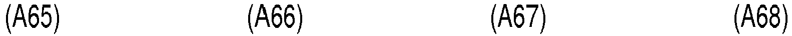 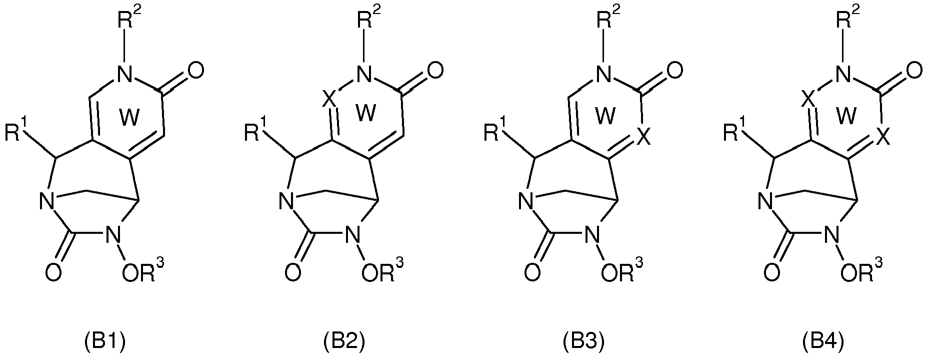 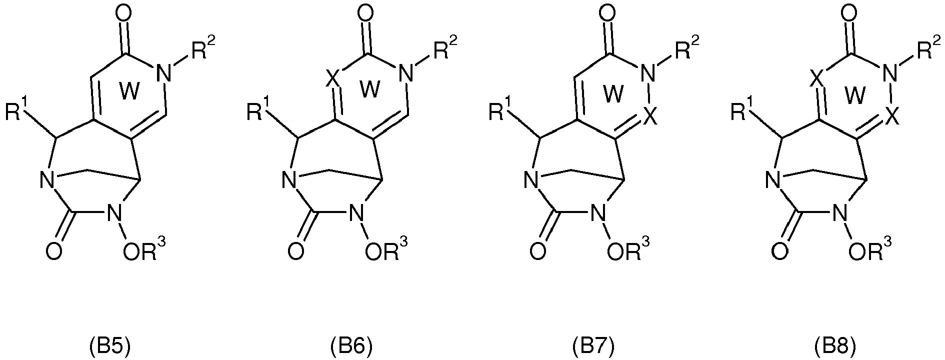 wherein W, unsubstituted or substituted by one or more T, X, R¹, R², R³ and T are defined according to formula (I).

More preferably, the compound according to the invention is selected from the compounds of formulae (I*), (A*), (B*) 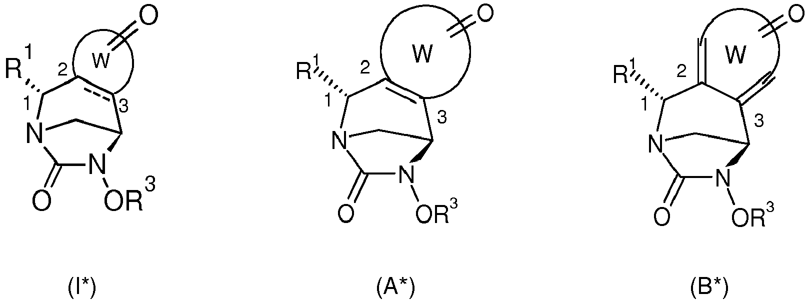 wherein W, unsubstituted or substituted by one or more T, X, n, R¹, R², R³ and T are defined according to formula (I).

The invention also provides compounds of formula (A1*) to (A68*) and (B1*) to (B8*) respectively corresponding to the stereoisomers of the compounds of formulae (A1) to (A68) and (B1) to (B8).

Preferred compounds according to the invention are compounds selected from compounds of formulae (I), (A), (B), (A1) to (A68), (B1) to (B8), (I*), (A*), (B*), (A1*) to (A68*) and (B1*) to (B8*) wherein W, unsubstituted or substituted by one or more T, represents a non-aromatic, unsaturated 5-membered heterocycle comprising a group N-R² and a group (X)ₙ.

Other preferred compounds according to the invention are compounds selected from compounds of formulae (I), (A), (A1) to (A68), (I*), (A*) and (A1*) to (A68*) wherein W, unsubstituted or substituted by one or more T, represents a non-aromatic, unsaturated 5-membered heterocycle comprising a group N-R² and a group (X)ₙ and wherein W, unsubstituted or substituted by one or more T, X, R¹, R², R³ and T are defined according to formula (I).

Other preferred compounds according to the invention are compounds selected from compounds of formulae (I), (A), (B), (A1) to (A68), (B1) to (B8), (I*), (A*), (B*), (A1*) to (A68*) and (B1*) to (B8*) wherein W, unsubstituted or substituted by one or more T, represents a non-aromatic, unsaturated 6-membered heterocycle comprising a group N-R² and a group (X)ₙ and wherein W, unsubstituted or substituted by one or more T, X, R¹, R², R³ and T are defined according to formula (I).

For the compounds according to the invention, R¹ preferably represents a carbon-linked, unsubstituted or substituted by one or more T¹, aromatic, saturated, totally or partially unsaturated 4-, 5- or 6-membered heterocycle comprising at least one nitrogen atom ; a hydrogen atom ; -CN ; -C(O)NHQ¹ ; -C(O)NHOQ¹ ; -C(O)NH-NHQ¹ ; -C(O)O-NHQ¹ ; - C(O)OQ¹ ; -(CH₂)OC(O)OQ¹ ; -(CH₂)₂OC(O)OQ¹ ; -(CH₂)OQ¹ ; -(CH₂)₂OQ¹ ; - (CH₂)OC(O)Q¹ ; -(CH₂)₂OC(O)Q¹ ; -(CH₂)-OC(O)NQ¹Q² ; -(CH₂)₂OC(O)NQ¹Q² ; - (CH₂)NHC(O)Q¹ ; -(CH₂)₂-NHC(O)Q¹ ; -(CH₂)NHS(O)₂Q¹ ; -(CH₂)₂NHS(O)₂Q¹ ; - (CH₂)NHS(O)₂NQ¹Q² ; -(CH₂)₂NHS(O)₂NQ¹Q² ; -(CH₂)NHC(O)OQ¹ ; -(CH₂)₂NHC(O)OQ¹ ; -(CH₂)NHC(O)NQ¹Q² ; -(CH₂)₂NHC(O)NQ¹Q². More preferably, R¹ represents a carbon-linked, unsubstituted or substituted by one or more T¹, aromatic, saturated, totally or partially unsaturated 4-, 5- or 6-membered heterocycle comprising at least one nitrogen atom ; -CN ; -C(O)NHQ¹ ; -C(O)NHOQ¹ ; -C(O)NH-NHQ¹ ; -(CH₂)₂OQ¹.

For the compounds according to the invention, R¹ represents equally preferably - (CH₂)NHQ³ ; -(CH₂)₂NHQ³ ; -(CH₂)NH-C(NHQ³)=NQ⁴ ; -(CH₂)₂NH-C(NHQ³)=NQ⁴ ; - (CH₂)NH-CH=NQ³ ; -(CH₂)₂NH-CH=NQ³ ; -C(NHQ³)=NQ⁴. More preferably, R¹ represents -(CH₂)NHQ³ ; -(CH₂)NH-C(NHQ³)=NQ⁴, in particular R¹ represents -(CH₂)NH₂ or - (CH₂)NH-C(NH₂)=NH.

For the compounds according to the invention, R¹ may represent a carbon-linked, unsubstituted or substituted by one or more T¹, aromatic, saturated, totally or partially unsaturated 4-, 5- or 6-membered heterocycle that comprises at least one nitrogen atom. Such a 4-, 5- or 6-membered heterocycle may further comprise one or more heteroatoms, for example 1, 2 or 3 further heteroatoms, preferably selected from N, O, S, S(O) or S(O)₂.

For the compounds according to the invention, R³ preferably represents SO₃H or CF₂COOH.

For the compounds according to the invention, Q¹ and Q² and the nitrogen atom to which they are bonded, may form together a saturated or partially unsaturated 4-, 5- or 6-membered heterocycle comprising 1, 2 or 3 heteroatoms. The resulting 4-, 5- or 6-membered heterocycle thus comprises the nitrogen atom bonded to Q¹ and Q² and one or two further optional heteroatoms.

For the compounds according to the invention, Q¹ and Q², identical or different, preferably represent H ; methyl ; -CH₂-CH₂-NH₂ ; -CH₂-CH₂-NH-CNH₂=NH ; -CH₂-CH₂-NH-CH=NH ; - CH₂-C(NH₂)=NH ; -CH₂-CH₂-OH ; -CH₂-CONH₂ ; a -(CH₂)ₚ-(saturated, partially or totally unsaturated or aromatic 4-, 5- or 6-membered heterocycle comprising at least one nitrogen atom) wherein the heterocycle can be substituted by one or more T², and p and T² are defined according to formula (I).

For the compounds according to the invention, Q¹ and Q², identical or different, more preferably represent H ; methyl ; -CH₂-CH₂-NH₂ ; -CH₂-CH₂-NH-CNH₂=NH ; -CH₂-CH₂-NH-CH=NH ; -CH₂-C(NH₂)=NH ; -CH₂-CH₂-OH ; -CH₂-CONH₂ ; a saturated, partially or totally unsaturated or aromatic 4-, 5- or 6-membered heterocycle comprising one nitrogen atom wherein the heterocycle can be substituted by one or more T² that is defined according to formula (I).

For the compounds according to the invention, Q³ and Q⁴, identical or different, preferably represent H or methyl.

For the compounds according to the invention, Q⁵ and Q⁶ and the nitrogen atom to which they are bonded, may form together a saturated or partially unsaturated 4-, 5- or 6-membered heterocycle comprising 1, 2 or 3 heteroatoms. The resulting 4-, 5- or 6-membered heterocycle thus comprises the nitrogen atom bonded to Q⁵ and Q⁶ and one or two further optional heteroatoms.

The term "alkyl", as used herein, refers to an aliphatic-hydrocarbon group which may be straight or branched, having 1 to 3 carbon atoms in the chain unless specified otherwise. Preferred alkyl groups have 1 or 2 carbon atoms in the chain. Specific examples of alkyl groups include, but are not limited to, methyl, ethyl, n-propyl, iso propyl. Preferably, the alkyl group is methyl or ethyl.

The term "fluoroalkyl", as used herein, refers to an alkyl group substituted with at least one fluorine atom. The term "alkyl" is as defined above. Specific examples of fluoroalkyl groups include but are not limited to trifluoromethyl, difluoromethyl, fluoromethyl.

The term "cycloalkyl" refers to a saturated monocyclic or bicyclic non-aromatic hydrocarbon ring of 3 to 6 carbon atoms, preferably 3 to 4 carbon atoms, which can comprise one or more unsaturation. Specific examples of monocyclic cycloalkyl groups include cyclopropyl, cyclobutyl, cyclopentyl, cyclohexyl. Preferably, the cycloalkyl group is cyclopropyl or cyclobutyl.

The term "fluorocycloalkyl" refers to a cycloalkyl group substituted with at least one fluorine atom. The term "cycloalkyl" is as defined above. Specific examples of fluorocycloalkyl groups include fluorocyclopropyl, difluorocyclopropyl, fluorocyclobutyl, difluorocyclobutyl.

The term "heterocycle", as used herein and without contrary definition specifically mentioned, either alone or in combination with another radical, refers to a monocyclic saturated, partially or totally unsaturated or aromatic hydrocarbon radical, preferably to a 4- to 10-membered hydrocarbon radical, comprising at least one heteroatom, such as N, O, S, S(O) or S(O)₂. Preferably, the heterocycle is a monocyclic saturated, partially or totally unsaturated or aromatic hydrocarbon radical, preferably a 4- to 6-membered hydrocarbon radical, comprising at least one nitrogen atom and at least one further heteroatom, such as N, O, S, S(O) or S(O)₂. The carbon atoms of the heterocycle can also be oxidized to form a C(O) group. Suitable heterocycles are also disclosed in the Handbook of Chemistry and Physics, 76th Edition, CRC Press, Inc., 1995-1996, pages 2-25 to 2-26. Examplary heterocycle groups include, but are not limited to, azetidinyl, oxetanyl, oxazolyl, oxazolidinyl, oxadiazolyl, pyrrolyl, pyrrolidinyl, pyridyl, tetrahydropyridinyl, piperidinyl, morpholinyl, pyrazolyl, pyrimidinyl, pyrazinyl, tetrazolyl, imidazolyl, thienyl, thiazolyl, furanyl, thiadiazolyl, isothiazolyl, triazolyl, tetrazolyl, pyrazolyl, isoxazolyl, 2-pyrrolidinonyl, imidazol-2,4-dione, 1,2,4-oxadiazol-5-one, 1,5-dihydropyrrolyl-2-one, pyrazinone, pyridazinone, pyridone, pyrimidone, dioxanyl, pyrrolidinyl, imidazolidinyl, pyranyl, tetrahydrofuranyl, dioxolanyl, tetrahydropyranyl. Preferably, in the compounds according to the invention, the heterocycle is linked to the structure of the compounds by a carbon atom of the heterocycle (also said carbon-linked heteroatom).

Moreover some compounds according to this invention may contain a basic amino group and thus may form an inner zwitterionic salt (or zwitterion) with the acidic group (R³) - OSO₃H, -OCFHCO₂H or -OCF₂CO₂H and such inner zwitterionic salts are also included in this invention.

The expression "optionally substituted" means "non-substituted or substituted by chemical groups that are further defined" or "unsubstituted or substituted chemical groups that are further defined".

The term "racemate" is employed herein to refer to an equal amount of two specific enantiomers.

The term "enantiomer" is employed herein to refer to one of the two specific stereoisomers which is a non-superimposable mirror image with one other but is related to one other by reflection.

The compounds according to the invention may include one or more asymmetric carbon atoms and may thus exist in the form of optical isomers as well as in the form of racemic or non-racemic mixtures thereof. The compounds according to the invention can be used as a single isomer or as a mixture of stereochemical isomeric forms. Diastereoisomers, *i.e.,* non-superimposable stereochemical isomers can be separated by conventional means such as chromatography, distillation, crystallization or sublimation. The optical isomers (enantiomers) can be obtained by using optically active starting materials, by resolution of the racemic mixtures according to conventional processes, for example by formation of diastereoisomeric salts by treatment with an optically active acid or base or by using chiral chromatography column.

As used herein, the expression "pharmaceutically acceptable salts" refers to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the present invention can be synthesized from the parent compound which comprises a basic or an acidic moiety, by conventional chemical methods. Furthermore, the expression "pharmaceutically acceptable salt" refers to relatively nontoxic, inorganic and organic acid or base addition salts of the compounds of the present invention. These salts can be prepared in situ during the final isolation and purification of the compounds. In particular, the acid addition salts can be prepared by separately reacting the purified compound in its purified form with an organic or inorganic acid and by isolating the salt thus formed. Among the examples of acid addition salts are the hydrobromide, hydrochloride, hydroiodide, sulfamate, sulfate, bisulfate, phosphate, nitrate, acetate, propionate, succinate, oxalate, valerate, oleate, palmitate, stearate, laurate, borate, benzoate, lactate, tosylate, citrate, maleate, fumarate, tartrate, naphthylate, mesylate, glucoheptanate, glucoronate, glutamate, lactobionate, malonate, salicylate, methylenebis-b-hydroxynaphthoate, gentisic acid, isethionate, di-p-toluoyltartrate, ethanesulfonate, benzenesulfonate, cyclohexyl sulfamate, quinateslaurylsulfonate salts, and the like. Examples of base addition salts include ammonium salts such as tromethamine, meglumine, epolamine, etc, metal salts such as sodium, lithium, potassium, calcium, zinc or magnesium salts with organic bases such as dicyclohexylamine salts, N-methyl-D-glucamine. Lists of suitable salts may be found in Remington's Pharmaceutical Sciences, 17th ed., Mack Publishing Company, Easton, PA, 1985, p. 1418, P.H. Stahl, C.G. Wermuth, Handbook of Pharmaceutical salts - Properties, Selection and Use, Wiley-VCH, 2002 and S.M. Berge et al. "Pharmaceutical Salts" J. Pharm. Sci, 66: p.1-19 (1977).

Compounds according to the invention also include isotopically-labeled compounds wherein one or more atoms is replaced by an atom having the same atomic number, but an atomic mass or mass number different from the atomic mass or mass number usually found in nature. Examples of isotopes suitable for inclusion in the compounds described above and are not limited to ²H, ³H, ¹¹C, ¹³C, ¹⁴C, ¹⁸F, ¹⁹F, ¹³N, ¹⁵N, ³³S, ³⁴S, ³⁵S, ³⁶S, ¹⁷O or ¹⁸O. Isotopically-labeled compounds are useful in drug and/or substrate tissue distribution studies. Substitution with heavier isotopes such as deuterium (²H) affords greater metabolic stability (for example increased *in vivo* half-life or reduced dosage requirements). Isotopically-labeled compounds are prepared by any suitable method or by processes using an appropriate isotopically-labeled reagent in replacement of the non-labeled reagent otherwise employed.

The invention provides compounds having antibacterial properties and/or compounds acting as β-lactamase inhibitors.

The invention also provides a process for the preparation of a compound according to the invention. In particular the invention provides a process for the preparation of compound selected from compounds of formulae (I), (A), (B), (A1) to (A68), (B1) to (B8), (I*), (A*), (B*), (A1*) to (A68*) and (B1*) to (B8*) according to the invention.

A particular process according to the invention is represented in scheme 1.

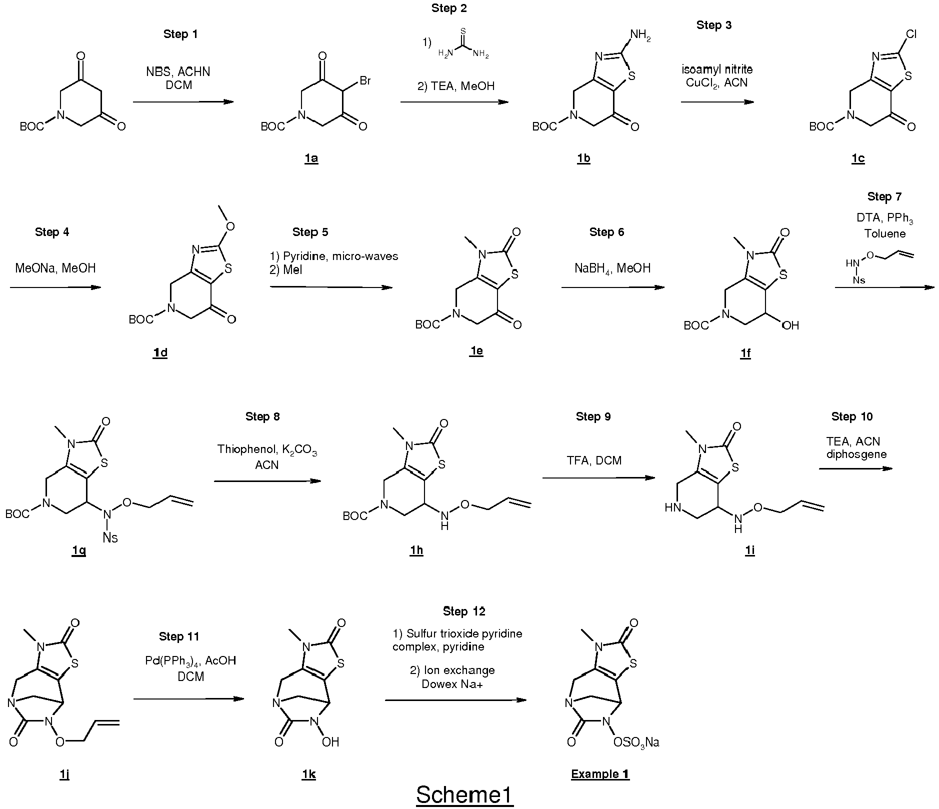

The processes of scheme 1 can be adapted for preparing further compounds according to the invention. Further processes for the preparation of compounds according to the invention can be derived from the process of scheme 1.

The invention also provides the use of the compounds according to the invention in the control of bacteria. The compound according to the invention is then usually used in combination with at least one pharmaceutically acceptable excipient.

The expression "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The present invention also provides a composition, preferably a pharmaceutical composition, comprising at least one compound according to the invention in mixture with a pharmaceutically acceptable excipient. The composition according to the invention may thus comprise at least one compound selected from compounds of formulae (I), (A), (B), (A1) to (A68), (B1) to (B8), (I*), (A*), (B*), (A1*) to (A68*) and (B1*) to (B8*) in mixture with a pharmaceutically acceptable excipient.

The term "pharmaceutically acceptable carrier" or "pharmaceutically acceptable excipient" is employed for any excipient, solvent, dispersion medium, absorption retardant, diluent or adjuvant etc., such as preserving or antioxidant agents, fillers, binders, disintegrating agents, wetting agents, emulsifying agents, suspending agents, solvents, dispersion media, coatings, antibacterial agents, isotonic and absorption delaying agents and the like, that does not produce a secondary reaction, for example an allergic reaction, in humans or animals. Typical, non-limiting examples of excipients include mannitol, lactose, magnesium stearate, sodium saccharide, talcum, cellulose, sodium croscarmellose, glucose, gelatin, starch, lactose, dicalcium phosphate, sucrose, kaolin, magnesium carbonate, wetting agents, emulsifying agents, solubilizing agents, sterile water, saline, pH buffers, non-ionic surfactants, lubricants, stabilizing agents, binding agents and edible oils such as peanut oil, sesame oils and the like. In addition, various excipients commonly used in the art may be included. Pharmaceutically acceptable carriers or excipients are well known to a person skilled in the art, and include those described in Remington's Pharmaceutical Sciences (Mack Publishing Company, Easton, USA, 1985), Merck Index (Merck & Company, Rahway, N.J.), Gilman et al (Eds. The pharmacological basis of therapeutics, 8th Ed., Pergamon press., 1990). Except insofar as any conventional media or adjuvant is incompatible with the active ingredient according to the invention, its use in the therapeutic compositions is contemplated.

The expression "antibacterial agent" as used herein, refers to any substance, compound or their combination capable of inhibiting, reducing or preventing growth of bacteria, inhibiting or reducing ability of bacteria to produce infection in a subject, or inhibiting or reducing ability of bacteria to multiply or remain infective in the environment, or decreasing infectivity or virulence of bacteria.

The antibacterial agent can be selected among the following families: aminoglycosides, beta-lactams, glycylcyclines, tetracyclines, quinolones, fluoroquinolones, glycopeptides, lipopeptides, macrolides, ketolides, lincosamides, streptogramins, oxazolidinones and polymyxins alone or in mixture. Preferably, the further antibacterial agent is selected among the beta-lactam families, and more preferably among penicillin, cephalosporins, penems, carbapenems and monobactam, alone or in mixture.

Among the penicillin the antibacterial agent is preferably selected in the group consisting of amoxicillin, ampicillin, azlocillin, mezocillin, apalcillin, hetacillin, bacampicillin, carbenicillin, sulbenicillin, temocillin, ticarcillin, piperacillin, mecillinam, pivmecillinam, methicillin, ciclacillin, talampacillin, aspoxicillin, oxacillin, cloxacillin, dicloxacillin, flucloxacillin, nafcillin, and pivampicillin, alone or in mixture.

Among the cephalosporin, the antibacterial agent is preferably selected in the group consisting of cefatriazine, cefazolin, cefoxitin, cephalexin, cephradine, ceftizoxime, cephacetrile, cefbuperazone, cefprozil, ceftobiprole, ceftobiprole medocaril, ceftaroline, ceftaroline fosaminyl, cefalonium, cefminox, ceforanide, cefotetan, ceftibuten, cefcapene pivoxil, cefditoren pivoxil, cefdaloxime cefroxadine, ceftolozane and S-649266, cephalothin, cephaloridine, cefaclor, cefadroxil, cefamandole, cefazolin, cephalexin, cephradine, ceftizoxime, cephacetrile, cefotiam, cefotaxime, cefsulodin, cefoperazone, cefmenoxime, cefmetazole, cephaloglycin, cefonicid, cefodizime, cefpirome, ceftazidime, ceftriaxone, cefpiramide, cefbuperazone, cefozopran, cefepime, cefoselis, cefluprenam, cefuzonam, cefpimizole, cefclidine, cefixime, ceftibuten, cefdinir, cefpodoxime axetil, cefpodoxime proxetil, cefteram pivoxil, cefetamet pivoxil, cefcapene pivoxil, cefditoren pivoxil, cefuroxime, cefuroxime axetil, loracarbef, and latamoxef, alone or in mixture. Among the carbapenem, the antibacterial agent is preferably selected in the group consisting of imipenem, doripenem, meropenem, biapenem, ertapenem and panipenem, alone or in mixture.

Among the monobactam, the antibacterial agent is preferably selected in the group consisting of aztreonam, tigemonam, carumonam, BAL30072 and nocardicin A, alone or in mixture.

The present invention also provides a kit comprising:
▪ a pharmaceutical composition according to the invention, and
▪ at least one other composition comprising one or more antibacterial agents, preferably at least one of these antibacterial agents is a beta-lactam.

The two compositions can each be prepared separately with one specific pharmaceutically acceptable carrier, and can then be mixed, especially extemporaneously.

The present invention also refers to a compound selected within the compounds of formulae (I), (A), (B), (A1) to (A68), (B1) to (B8), (I*), (A*), (B*), (A1*) to (A68*) and (B1*) to (B8*) according to the invention for its use as a medicine.

The present invention also refers to a compound selected within the compounds of formulae (I), (A), (B), (A1) to (A68), (B1) to (B8), (I*), (A*), (B*), (A1*) to (A68*) and (B1*) to (B8*) according to the invention for its use for the preparation of a medicine.

The present invention also refers to a compound selected within the compounds of formulae (I), (A), (B), (A1) to (A68), (B1) to (B8), (I*), (A*), (B*), (A1*) to (A68*) and (B1*) to (B8*) according to the invention for its use as an antibacterial agent.

The present invention also refers to the use of a compound selected within the compounds of formulae (I), (A), (B), (A1) to (A68), (B1) to (B8), (I*), (A*), (B*), (A1*) to (A68*) and (B1*) to (B8*) according to the invention or to the use of a pharmaceutical composition according to the invention for the preparation of an antibacterial agent comprising medicine.

The present invention also refers to the use of a compound selected within the compounds of formulae (I), (A), (B), (A1) to (A68), (B1) to (B8), (I*), (A*), (B*), (A1*) to (A68*) and (B1*) to (B8*) according to the invention or to the use of a pharmaceutical composition according to the invention for the preparation of a beta-lactamase inhibitor comprising medicine.

The present invention also refers to the use of a compound selected within the compounds of formulae (I), (A), (B), (A1) to (A68), (B1) to (B8), (I*), (A*), (B*), (A1*) to (A68*) and (B1*) to (B8*) according to the invention or to the use of a pharmaceutical composition according to the invention for the preparation of a medicine comprising an antibacterial agent and a beta-lactamase inhibitor.

The present invention also refers to the use of a compound selected within the compounds of formulae (I), (A), (B), (A1) to (A68), (B1) to (B8), (I*), (A*), (B*), (A1*) to (A68*) and (B1*) to (B8*) according to the invention or to the use of a pharmaceutical composition according to the invention or to the use of a kit according to the invention for the treatment or for the prevention of at least one bacterial infection.

The present invention also refers to the use of a compound selected within the compounds of formulae (I), (A), (B), (A1) to (A68), (B1) to (B8), (I*), (A*), (B*), (A1*) to (A68*) and (B1*) to (B8*) according to the invention or to the use of a pharmaceutical composition according to the invention or to the use of a kit according to the invention for the preparation of a medicine useful in the treatment or in the prevention of at least one bacterial infection.

The terms "prevention", "prevent" and "preventing" as used herein are intended to mean the administration of a compound or composition according to the invention in order to prevent infection by bacteria or to prevent occurrence of related infection and/or diseases. The terms "prevention", "prevent" and "preventing" also encompass the administration of a compound or composition according to the present invention in order preventing at least one bacterial infection, by administration to a patient susceptible to be infected, or otherwise at a risk of being infected by this bacteria.

The terms "treatment", "treat" and "treating" as used herein are intended to mean in particular the administration of a treatment comprising a compound or composition according to the invention to a patient suffering from an infection. The terms "treatment", "treat" and "treating" as used herein, also refer to administering a compound or composition according to the invention, optionally in combination with one or more further antibacterial agent, in order:
▪ to reduce or to eliminate either bacterial infection or one or more symptoms associated with a bacterial infection, or
▪ to retard the progression of a bacterial infection or of one or more symptoms associated with a bacterial infection, or
▪ to reduce the severity of a bacterial infection or of one or more symptoms associated with a bacterial infection, or
▪ to suppress the clinical manifestation of a bacterial infection, or
▪ to suppress the manifestation of adverse symptoms caused by a bacterial infection.

The expression "infection" or "bacterial infection" as used herein, include the presence of bacteria, in or on a subject, which, if its growth were inhibited, would result in a benefit to the subject. As such, the term "infection" or "bacterial infection" in addition to referring to the presence of bacteria also refer to normal flora, which is not desirable. The term "infection" includes infection caused by bacteria. Examples of such bacterial infections are urinary tract infection (UTI), kidney infections (pyelonephritis), gynecological and obstetrical infections, respiratory tract infection (RTI), acute exacerbation of chronic bronchitis (AECB), Community-acquired pneumonia (CAP), hospital-acquired pneumonia (HAP), ventilator associated pneumonia (VAP), intra-abdominal pneumonia (IAI), acute otitis media, acute sinusitis, sepsis, catheter-related sepsis, chancroid, chlamydia, skin infections, bacteremia.

The term "growth" as used herein, refers to the growth of one or more microorganisms and includes reproduction or population expansion of a microorganism, such as bacteria. The term also includes maintenance of on-going metabolic processes of a microorganism, including processes that keep the microorganism alive.

According to the invention, bacteria are chosen amongst gram-positive bacteria or gram-negative bacteria, preferably gram-negative bacteria. According to the invention, bacteria can be also chosen among bacteria producing "beta-lactamase" or "β-lactamase". These bacteria are well known by the person skilled in the art. The term "beta-lactamase" or "β-lactamase" as used herein, refers to any enzyme or protein or any other substance that is able to break down a beta-lactam ring. The term "beta-lactamase" or "β-lactamase" includes enzymes that are produced by bacteria and that have the ability to hydrolyze, either partially or completely, the beta-lactam ring present in a compound such as an antibacterial agent.

Among the gram-positive bacteria, the bacteria according to the invention is preferably chosen among *Staphylococcus, Streptococcus, Staphylococcus species* (including *Staphylococcus aureus, Staphylococcus epidermidis*), *Streptococcus species* (including *Streptococcus pneumonia, Streptococcus agalactiae*), *Enterococcus species* (including *Enterococcus faecalis* and *Enterococcus faecium*)*.*

Among the gram-negative bacteria, the bacteria according to the invention is preferably chosen among *Acinetobacter* species (including *Acinetobacter baumannii*), *Citrobacter* species, *Escherichia* species (including *Escherichia coli*), *Haemophilus influenza, Morganella morganii, Klebsiella* species (including *Klebsiella pneumonia*), *Enterobacter* species (including *Enterobacter cloacae*), *Neisseria gonorrhoeae, Burkholderia* species (including *Burkholderia cepacia*), (*Proteus* species (including *Proteus mirabilis*), *Serratia* species (including *Serratia marcescens*), *Pseudomonas aeruginosa.*

The invention thus preferably refers to a compound selected within the compounds of formulae (I), (A), (B), (A1) to (A68), (B1) to (B8), (I*), (A*), (B*), (A1*) to (A68*) and (B1*) to (B8*) according to the invention or to a pharmaceutical composition according to the invention or to a kit according to the invention for its use for the treatment or for the prevention of a bacterial infection, preferably caused by bacteria producing one or more beta-lactamases. Preferably, the bacteria are chosen amongst gram-positive bacteria or gram-negative bacteria, more preferably gram-negative bacteria.

The present invention also refers to the use of a compound selected within the compounds of formulae (I), (A), (B), (A1) to (A68), (B1) to (B8), (I*), (A*), (B*), (A1*) to (A68*) and (B1*) to (B8*) according to the invention or to a pharmaceutical composition according to the invention for the preparation of a medicine for the treatment or for the prevention of a bacterial infection, preferably caused by bacteria producing one or more beta-lactamases. Preferably, the bacteria are chosen amongst gram-positive bacteria or gram-negative bacteria, more preferably gram-negative bacteria.

The present invention also refers to a kit according to the invention, for its simultaneous, separated or sequential administration to a patient in need thereof in the treatment or in the prevention of bacterial infections, preferably caused by bacteria producing one or more beta-lactamases. Preferably, the bacteria are chosen amongst gram-positive bacteria or gram-negative bacteria, more preferably gram-negative bacteria.

The present invention also refers to a compound selected within the compounds of formulae (I), (A), (B), (A1) to (A68), (B1) to (B8), (I*), (A*), (B*), (A1*) to (A68*) and (B1*) to (B8*) according to the invention for its use in combination with one or more further antibacterial agents, preferably at least one of the further antibacterial agents being a beta lactam compound, for the treatment or for the prevention of bacterial infections, preferably caused by bacteria producing one or more beta-lactamases. Preferably, the bacteria are chosen amongst gram-positive bacteria or gram-negative bacteria, more preferably gram-negative bacteria, and wherein a compound selected within the compounds of formulae (I), (A), (B), (A1) to (A68), (B1) to (B8), (I*), (A*), (B*), (A1*) to (A68*) and (B1*) to (B8*) according to the invention and the further antibacterial agent are administered simultaneously, separately or sequentially.

The present invention also refers to the use of a compound selected within the compounds of formulae (I), (A), (B), (A1) to (A68), (B1) to (B8), (I*), (A*), (B*), (A1*) to (A68*) and (B1*) to (B8*) according to the invention or of a pharmaceutical composition according to the invention or of a kit according to the invention for the prevention or for the treatment of bacterial infections, preferably of a bacterial infection, preferably caused by bacteria producing one or more beta-lactamases. Preferably, the bacteria are chosen amongst gram-positive bacteria or gram-negative bacteria, more preferably gram-negative bacteria.

The present invention also relates to a method for the treatment or prevention of bacterial infections, preferably caused by bacteria producing one or more beta-lactamases comprising the administration of a therapeutically effective amount of a compound selected within the compounds of formulae (I), (A), (B), (A1) to (A68), (B1) to (B8), (I*), (A*), (B*), (A1*) to (A68*) and (B1*) to (B8*) according to the invention, or of a pharmaceutical composition according to the invention or of a kit according to the invention to a patient in need thereof. Preferably, the bacteria are chosen amongst gram-positive bacteria or gram-negative bacteria, more preferably gram-negative bacteria.

The term "patient" means a person or an animal at risk of being infected by bacteria or, a person or an animal being infected by bacteria, preferably by gram-positive and by gram-negative bacteria, more preferably by gram-negative bacteria. As used herein, the term "patient" refers to a warm-blooded person or animal such as a mammal, preferably a human or a human child, who is afflicted with, or has the potential to be afflicted with one or more infections and conditions described herein. The identification of those subjects who are in need of treatment of herein-described diseases and conditions is well within the ability and knowledge of one skilled in the art. A veterinarian or a physician skilled in the art can readily identify, by the use of clinical tests, physical examination, medical or family history or biological and diagnostic tests, those subjects who are in need of such a treatment.

The expression "therapeutically effective amount" or "pharmaceutically effective amount" as used herein, refer to an amount of a compound according to the invention, which when administered to a patient in need thereof, is sufficient to effect treatment for disease-states, conditions, or disorders for which the compound has utility. Such an amount would be sufficient to elicit the biological or medical response of a tissue system, or patient that is sought by a researcher or a clinician. The amount of a compound according to the invention which constitutes a "therapeutically effective amount" will vary, notably depending on the compound itself and its biological activity, the composition used for administration, the time of administration, the route of administration, the rate of excretion of the compound, the duration of the treatment, the type of disease-state or disorder being treated and its severity, drugs used in combination with or coincidentally with the compounds of the invention, and the age, body weight, general health, sex and diet of the patient. Such a "therapeutically effective amount" can be determined by one of ordinary skilled in the art having regard to its own knowledge, and this disclosure. Preferably, the compound according to the invention is administered in an amount comprised between 0.1 to 30 g per day.

The compound according to the invention may be provided in an aqueous physiological buffer solution for parenteral administration. The compound of the present invention is also capable of being administered in unit dose forms, wherein the expression "unit dose" means a single dose which is capable of being administered to a patient, and which can be readily handled and packaged, remaining as a physically and chemically stable unit dose comprising either the active compound itself, or as a pharmaceutically acceptable composition, as described herein. The compound provided herein can be formulated into pharmaceutical compositions by admixture with one or more pharmaceutically acceptable excipients. Such unit dose compositions may be prepared for use by oral administration, particularly in the form of tablets, simple capsules or soft gel capsules; or intranasally, particularly in the form of powders, nasal drops, or aerosols; or dermally, for example, topically in ointments, creams, lotions, gels or sprays, or via trans-dermal patches.

The pharmaceutical composition may be conveniently administered in unit dosage form and may be prepared by any method well-known in the pharmaceutical art, for example, as described in Remington: The Science and Practice of Pharmacy, 20th ed.; Gennaro, A. R., Ed.; Lippincott Williams & Wilkins: Philadelphia, PA, 2000.

Preferred formulations include pharmaceutical compositions wherein a compound according to the present invention is formulated for oral or parenteral administration.

For oral administration, tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings and flavorings. In addition, the active compounds may be incorporated into fast dissolved, modified-release or sustained-release preparations and formulations, and wherein such sustained-release formulations are preferably bi-modal. Preferred tablets contain lactose, cornstarch, magnesium silicate, croscarmellose sodium, povidone, magnesium stearate or talc in any combination.

Liquid preparations for parenteral administration include sterile aqueous or non-aqueous solutions, suspensions and emulsions. The liquid compositions may also include binders, buffers, preservatives, chelating agents, sweetening, flavoring and coloring agents, and the like. Non-aqueous solvents include alcohols, propylene glycol, polyethylene glycol, vegetable oils such as olive oil, and organic esters such as ethyl oleate. Aqueous carriers include mixtures of alcohols and water, buffered media, and saline. In particular, biocompatible, biodegradable lactide polymer, lactide/glycolide copolymer, or polyoxyethylene-polyoxypropylene copolymers may be useful excipients to control the release of the active compound. Intravenous vehicles can include fluid and nutrient replenishers, electrolyte replenishers, such as those based on Ringer's dextrose, and the like. Other potentially useful parenteral delivery systems for the active compound include ethylene-vinyl acetate copolymer particles, osmotic pumps, implantable infusion systems and liposomes.

Alternative modes of administration include formulations for inhalation, which include such means as dry powder, aerosol, or drops. They may be aqueous solutions comprising, for example, polyoxyethylene-9-lauryl ether, glycocholate and deoxycholate, or oily solutions for administration in the form of nasal drops, or as a gel to be applied intranasally. Formulations for buccal administration include, for example, lozenges or pastilles and may also include a flavored base, such as sucrose or acacia, and other excipients such as glycocholate. Formulations suitable for rectal administration are preferably presented as unit-dose suppositories, with a solid based carrier, and may include a salicylate. Formulations for topical application to the skin preferably take the form of an ointment, cream, lotion, paste, gel, spray, aerosol, or oil. Carriers which can be used include petroleum jelly, lanolin, polyethylene glycols, alcohols, or their combinations.

Formulations suitable for transdermal administration can be presented as discrete patches and can be lipophilic emulsions or buffered, aqueous solutions, dissolved and/or dispersed in a polymer or an adhesive.

The invention is further illustrated but not restricted by the description of the following examples.

### Examples

The following examples are provided for the purpose of illustrating the present invention and by no means should be interpreted to limit the scope of the present invention.

The first part represents the preparation of the compounds according to the invention (intermediates and final compounds) whereas the second part describes the evaluation of antibacterial activity of compounds according to the invention.

### Preparation of the compounds and biological activity:

### Abbreviations or symbols used herein include:

- ACHN:: 1,1'-azobis(cyclohexanecarbonitrile)
- ACN:: acetonitrile
- AcOH:: acetic acid
- Bn:: benzyl
- Boc:: *tert*-butoxycarbonyl
- Boc₂O:: *tert*-butoxycarbonyl anhydride
- BocON:: [2-(*tert*-butoxycarbonyloxyimino)-2-phenylacetonitrile]
- bs:: broad singlet
- Burgess reagent:: methyl *N*-(triethylammoniosulfonyl)carbamate
- CFU:: colony-forming units
- CLSI:: clinical laboratory standards institute
- d:: doublet
- DBU:: 1,8-diazabicyclo[5.4.0]undec-7-ene
- DCM:: dichloromethane
- dd:: double doublet
- ddd :: double double doublet
- ddt :: double double triplet
- dq:: double quartet
- dt :: double triplet
- DTA:: di-*tert*-butylazodicarboxylate
- DEAD:: diethyl azodicarboxylate
- Dess-Martin periodinane:: 1,1,1-tris(acetyloxy)-1,1-dihydro-1,2-benziodoxol-3-(1*H*)-one
- DIAD:: diisopropyl azodicarboxylate
- DIPEA:: *N,N*-diisopropylethylamine
- DMAP:: 4-dimethylaminopyridine
- DMF:: *N,N*-dimethylformamide
- DMSO:: dimethylsulfoxide
- EtOAc:: ethyl acetate
- Et₂O:: diethyl ether
- h:: hours
- HATU:: 1-[Bis(dimethylamino)methylene]-1*H*-1,2,3-triazolo[4,5-*b*]pyridinium-3-oxid hexafluorophosphate
- m :: multiplet
- min:: minutes
- MeOH:: methanol
- MeONa:: sodium methoxide
- MIC:: minimum inhibitory concentration
- MS:: mass spectrometry
- MsCl:: methanesulfonyl chloride
- NBS:: *N*-bromosuccinimide
- NMR:: nuclear magnetic resonance spectroscopy
- Ns:: nosyl, nitrobenzenesulfonyl
- Pd(Ph₃)₄:: tetrakis(triphenylphosphine)palladium(0)
- PG:: protective group
- PhSH:: thiophenol
- PMe₃:: trimethylphosphine
- PPh₃:: triphenylphosphine
- Ppm:: parts per million
- q:: quartet
- rt:: room temperature
- s:: singlet
- SEM:: [2-(trimethylsilyl)ethoxy]methyl
- t:: triplet
- TBAF:: tetra-*n*-butylammonium fluoride
- TBDMSOTf:: trifluoromethanesulfonic acid tert-butyldimethylsilyl ester
- TBSOTf:: trimethylsilyl trifluoromethanesulfonate
- tBuOK:: potassium *tert*-butoxide
- TEA:: trimethylamine
- TFA:: trifluoroacetic acid
- THF:: tetrahydrofuran
- THP:: tetrahydropyranyl
- TLC:: thin layer chromatography
- TMSI:: Iodotrimethylsilane

### Example 1: synthesis of mono-(5-methyl-4,9-dioxo-3-thia-5,8,10-triaza tricyclo[6.2.1.0^{2,6}] undec-2(6)-en-10-yl) ester sulfonate sodium salt

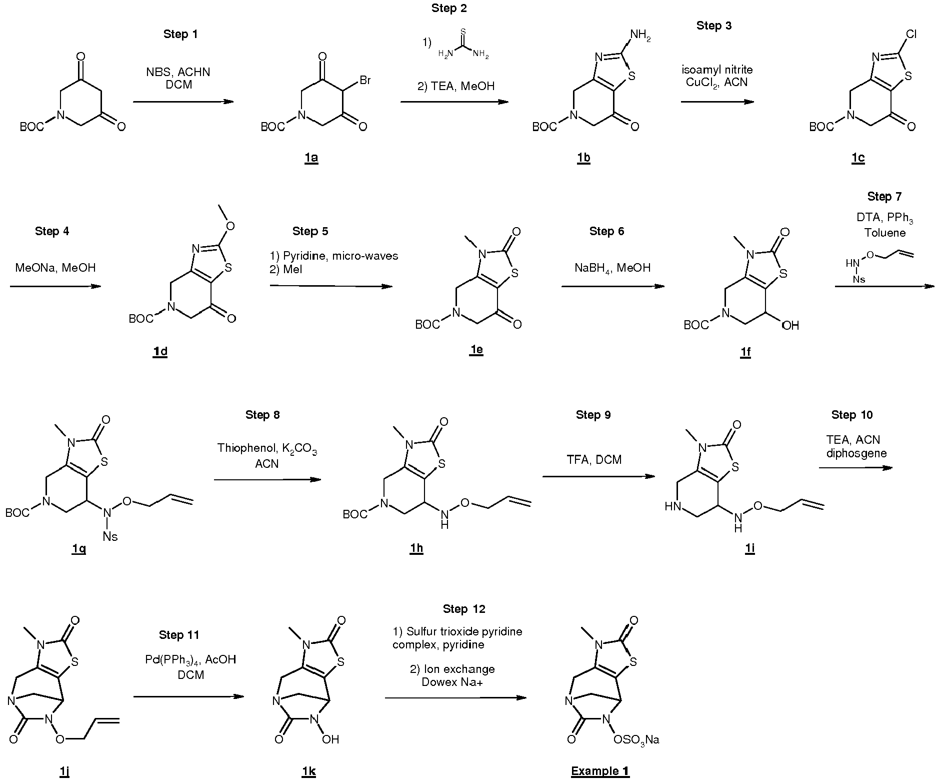

### Step 1: Preparation of intermediate tert-butyl 4-bromo-3,5-dioxo-piperidine-1-carboxylate (1a)

To a solution of *tert*-butyl 3,5-dioxopiperidine-1-carboxylate (3 g, 14.07 mmol) in anhydrous DCM (60 mL) under inert atmosphere at 0°C was successively added NBS (2.5 g, 14,07 mmol) and ACHN (0.223 g, 0.91 mmol). The reaction mixture was stirred 2 h at 0°C. The solution was washed with water, then with NaCl aqueous solution. The organic layer was dried over Na₂SO₄ and evaporated *in vacuo* to give *tert*-butyl 4-bromo-3,5-dioxo-piperidine-1-carboxylate (1a) (4.11 g, 14.07 mmol, quantitative yield) as an off-white solid.

MS *m*/*z* ([M+H-*tert*butyl]⁺) 236/238.

MS *m*/*z* ([M-H]⁻) 290/292.

¹H NMR (400 MHz, CDCl₃): *δ* (ppm) 1.48 (s, 9H), 4.35 (bs, 4H).

### Step 2: Preparation of intermediate tert-butyl 2-amino-7-oxo-6,7-dihydro-4H-thiazolo[4,5-c]pyridine-5-carboxylate (1b)

To a solution of *tert-*butyl 4-bromo-3,5-dioxo-piperidine-1-carboxylate (1a) (0.500 g, 1.71 mmol) in anhydrous MeOH (8 mL) under inert atmosphere was added thiourea (0.226 g, 3.42 mmol). After stirring 30 min at rt, TEA (0.477 mL, 3.42 mmol) was added and the mixture was refluxed 5 h. MeOH was evaporated and the residue was solubilized with EtOAc. The solution was washed with water, 10% of Na₂CO₃ aqueous solution and NaCl aqueous solution. The organic phase was dried over Na₂SO₄, filtered and evaporated. The solid was triturated with cyclohexane and filtered to give *tert-*butyl 2-amino-7-oxo-6,7-dihydro-4*H*-thiazolo[4,5-c]pyridine-5-carboxylate (1 b) (0.350 g, 1.30 mmol, 76%) as an off white solid.

MS *m*/*z* ([M+H]⁺) 270.

MS *m*/*z* ([M-H]⁻) 268.

¹H NMR (400 MHz, CDCl₃): *δ* (ppm) 1.48 (s, 9H), 4.23 (s, 2H), 4.66 (s, 2H), 5.77 (bs, 2H).

### Step 3: Preparation of intermediate tert-butyl 2-chloro-7-oxo-4,6-dihydrothiazolo[4,5-c]pyridine-5-carboxylate (1c)

To a solution of *tert*-butyl 2-amino-7-oxo-6,7-dihydro-4*H*-thiazolo[4,5-c]pyridine-5-carboxylate (1b) (0.473 g, 1.76 mmol) in anhydrous ACN (33 mL) under inert atmosphere at -20°C was added isoamyl nitrite (0.710 mL, 5.27 mmol). After 10 min at -20°C, Copper(II) chloride (0.473 g, 3.52 mmol) was added. The mixture was stirred for 1 h at - 20°C, then 4 h at rt. The solution was extracted with DCM, washed with 10% of Na₂CO₃ aqueous solution. The organic phase was dried over Na₂SO₄, filtered and evaporated. The product was purified by flash chromatography on silica gel (DCM/EtOAc 98/2) to give *tert*-butyl 2-chloro-7-oxo-4,6-dihydrothiazolo[4,5-c]pyridine-5-carboxylate (1c) (0.458 g, 1.59 mmol, 90%) as an off-white solid.

MS *m*/*z* ([M+H-*tert*butyl]⁺) 233/235.

MS *m*/*z* ([M-H]⁻) 287/289.

¹H NMR (400 MHz, CDCl₃): *δ* (ppm) 1.48 (s, 9H), 4.31 (s, 2H), 4.83 (s, 2H).

### Step 4: Preparation of intermediate tert-butyl 2-methoxy-7-oxo-6,7-dihydro-4H-thiazolo[4,5-c]pyridine-5-carboxylate (1d)

To a solution of *tert*-butyl 2-chloro-7-oxo-4,6-dihydrothiazolo[4,5-c]pyridine-5-carboxylate (1c) (3.75 g, 12.98 mmol) in anhydrous MeOH (97 mL) under inert atmosphere at -78°C was added dropwise a MeONa solution 0.5M (28.6 mL, 14.30 mmol). The reaction mixture was stirred for 15 min at -78°C, then for 30 min at rt. MeOH was removed under vacuum and the resulting residue was diluted with DCM and filtered on a mixture of silica gel and celite. The product was eluted with DCM/EtOAc 8/2, concentrated *in vacuo* and purified by flash chromatography on silica gel (cyclohexane/EtOAc 90/10 to 80/20) to provide *tert-*butyl 2-methoxy-7-oxo-6,7-dihydro-4*H*-thiazolo[4,5-c]pyridine-5-carboxylate (1d) (2.23 g, 7.84 mmol, 60%) as a yellow solid.

MS *m*/*z* ([M+H]⁺) 285.

¹H NMR (400 MHz, CDCl₃): *δ* (ppm) 1.49 (s, 9H), 4.17 (s, 3H), 4.26 (s, 2H), 4.70 (s, 2H).

### Step 5: Preparation of intermediate tert-butyl 3-methyl-2,7-dioxo-2,3,6,7-tetrahydro-4H-thiazolo[4,5-c]pyridine-5-carboxylate (1e)

*Tert*-butyl 2-methoxy-7-oxo-6,7-dihydro-4*H*-thiazolo[4,5-c]pyridine-5-carboxylate (1 d) (1.92 g, 6.74 mmol) was solubilized in pyridine (1.1 mL, 13.49 mmol) and warmed 30 min at 90°C under microwave irradiation. The mixture was evaporated and the residue was diluted with DCM (57 mL) at 0°C. A solution of Mel (0.840 mL, 13.49 mmol) in DCM (15 mL) was added dropwise to the reaction mixture. After stirring 1 h at 0°C and 2 h at rt, the precipitate was filtered. The filtrate was evaporated, solubilized in EtOAc and filtrated on silica gel cake to provide *tert*-butyl 3-methyl-2,7-dioxo-2,3,6,7-tetrahydro-4*H-*thiazolo[4,5-c]pyridine-5-carboxylate (1e) (1.63 g, 5.72 mmol, 85%).

MS *m*/*z* ([M+H]⁺) 285.

MS *m*/*z* ([M-H]⁻) 283.

¹H NMR (400 MHz, CDCl₃): *δ* (ppm) 1.49 (s, 9H), 3.36 (s, 3H), 4.26 (s, 2H), 4.61 (s, 2H).

### Step 6: Preparation of intermediate tert-butyl 7-hydroxy-3-methyl-2-oxo-2,3,6,7-tetrahydro-4H-thiazolo[4,5-c]pyridine-5-carboxylate (1f)

To a solution of *tert*-butyl 3-methyl-2,7-dioxo-2,3,6,7-tetrahydro-4*H*-thiazolo[4,5-c]pyridine-5-carboxylate (1e) (1.78 g, 6.25 mmol) in anhydrous MeOH (62 mL) at 0°C under inert atmosphere was added sodium borohydride (0.236 g, 6.25 mmol) by portions. The reaction mixture was stirred for 1 h and then concentrated under vacuum. The residue was diluted with EtOAc and filtered on silica gel cake to provide *tert*-butyl 7-hydroxy-3-methyl-2-oxo-2,3,6,7-tetrahydro-4*H-*thiazolo[4,5-c]pyridine-5-carboxylate (1f) (1.56 g, 5.45 mmol, 87%) as a yellow solid.

MS *m*/*z* ([M+H]⁺) 287.

¹H NMR (400 MHz, CDCl₃): *δ* (ppm) 1.50 (s, 9H), 3.22 (s, 3H), 3.55 (dd, J= 3.0/13.6 Hz, 1 H), 3.97-4.01 (m, 1 H), 4.04 (bs, 1 H), 4.52 (bs, 2H).

### Step 7: Preparation of intermediate tert-butyl 7-[allyloxy-(2-nitro-benzenesulfonyl)-amino]-3-methyl-2-oxo-2,3,6,7-tetrahydro-4H-thiazolo[4,5-c]pyridine-5-carboxylate (1g)

To a solution of *tert*-butyl 7-hydroxy-3-methyl-2-oxo-2,3,6,7-tetrahydro-4*H*-thiazolo[4,5-c]pyridine-5-carboxylate (1f) (1.56 g, 5.45 mmol) in anhydrous toluene (60 mL) under inert atmosphere was added *N-*allyloxy-2-nitro-benzenesulfonamide (1.41 g, 5.45 mmol) and Ph₃P (1.43 g, 5.45 mmol). DTA (1.42 g, 6.16 mmol) was added by portion and the mixture was stirred for 4 h at rt. The solution was filtered and concentrated under vacuum. The residue was purified by flash chromatography on silica gel (DCM/cyclohexane 70/30 to DCM/MeOH 99/1) to give *tert*-butyl 7-[allyloxy-(2-nitro-benzenesulfonyl)-amino]-3-methyl-2-oxo-2,3,6,7-tetrahydro-4*H*-thiazolo[4,5-c]pyridine-5-carboxylate (1g) (2.39 g, 4.53 mmol, 83%) as a yellow solid.

MS *m*/*z* ([M+H]⁺) 527.

### Step 8: Preparation of intermediate tert-butyl 7-allyloxyamino-3-methyl-2-oxo-2,3,6,7-tetrahydro-4H-thiazolo[4,5-c]pyridine-5-carboxylate (1h)

To a solution of *tert*-butyl 7-[allyloxy-(2-nitro-benzenesulfonyl)-amino]-3-methyl-2-oxo-2,3,6,7-tetrahydro-4*H*-thiazolo[4,5-c]pyridine-5-carboxylate (1g) (0.500 g, 0.95 mmol) in anhydrous ACN (6 mL) under inert atmosphere was added successively PhSH (0.487 mL, 4.75 mmol) and K₂CO₃ (0.985 g, 7.12 mmol). The reaction mixture was stirred for 16 h at rt. The reaction mixture was concentrated under vacuum, diluted with DCM and filtered to eliminate salts. The residue was purified by flash chromatography on silica gel (DCM/MeOH 100/0 to 95/5) then purified by preparative TLC (DCM/MeOH 95/5) to provide *tert-*butyl 7-allyloxyamino-3-methyl-2-oxo-2,3,6,7-tetrahydro-4*H*-thiazolo[4,5-c]pyridine-5-carboxylate (1 h) (0.289 g, 0.84 mmol, 89%).

MS *m*/*z* ([M+H]⁺) 342.

¹H NMR (400 MHz, CDCl₃): *δ* (ppm) 1.47 (s, 9H), 3.21 (s, 3H), 3.30-3.40 (m, 1H), 3.81-4.06 (m, 2H), 4.17-4.26 (m, 2H), 4.35-4.69 (m, 1 H), 5.16-5.32 (m, 2H), 5.38-5.43 (m, 1 H), 5.87-5.97 (m, 1H), 6.21 (bs, 1H).

### Step 9: Preparation of intermediate 7-allyloxyamino-3-methyl-4,5,6,7-tetrahydro-3H-thiazolo[4,5-c]pyridin-2-one (1i)

To a solution of 7-allyloxyamino-3-methyl-2-oxo-2,3,6,7-tetrahydro-4*H*-thiazolo[4,5-c]pyridine-5-carboxylic acid tert-butyl ester (1 h) (0.289 g, 0.85 mmol) in anhydrous DCM (5 mL) under inert atmosphere was added TFA (1 mL, 12.7 mmol). After stirring for 2 h at rt, the solution was cooled at 0°C and neutralized to pH 8 with NH₄OH solution 28%. The solution was diluted with water, the organic layer was separated from the aqueous, dried over Na₂SO₄, filtered and concentrated *in vacuo* to 7-allyloxyamino-3-methyl-4,5,6,7-tetrahydro-3*H*-thiazolo[4,5-c]pyridin-2-one (1 i) (0.140 g, 0.58 mmol, 68%) as a yellow oil.

¹H NMR (400 MHz, CDCl₃): *δ* (ppm) 2.97 (dd, J= 3.6/13.6 Hz, 1 H), 3.17 (s, 3H), 3.30 (dd, *J=* 2.8/13.6 Hz, 1 H), 3.59 (d, *J* = 1.5/16.5 Hz, 1 H), 3.69 (d, *J* = 1.0/16.3 Hz, 1 H), 3.75 (bs, 1 H), 4.20 (dt, *J* = 1.2/6.0 Hz, 2H), 5.20-5.25 (m, 2H), 5.61 (bs, 1 H), 5.88-5.97 (m, 1 H).

### Step 10: Preparation of intermediate 10-allyloxy-5-methyl-3-thia-5,8,10-triazatricyclo[6.2.1.0^{2,6}]undec-2(6)-ene-4,9-dione (1i)

To a solution of 7-allyloxyamino-3-methyl-4,5,6,7-tetrahydro-3*H*-thiazolo[4,5-c]pyridin-2-one (1i) (0.140 g, 0.58 mmol) in anhydrous ACN (90 mL) at -10°C under inert atmosphere was added TEA (0.323 mL, 2.32 mmol). A solution of diphosgene (0.035 mL, 0.29 mmol) in ACN (11 mL) was added dropwise at -10°C. After 2 h at -10°C then 18 h at rt, the mixture reaction was bubbled 30 min under nitrogen and concentrated under vacuum. The residue was diluted with DCM, washed with water and NaCl aqueous solution. The organic layer was dried over Na₂SO₄, filtered and concentrated *in vacuo.* The product was purified by preparative TLC (DCM/MeOH 96/4) to provide 10-allyloxy-5-methyl-3-thia-5,8,10-triaza-tricyclo[6.2.1.0^{2,6}] undec-2(6)-ene-4,9-dione (1j) (0.045 g, 0.17mmol, 29%) as an colourless oil.

MS *m*/*z* ([M+H]⁺) 268.

¹H NMR (400 MHz, CDCl₃): *δ* (ppm) 3.16 (s, 3H), 3.28 (dd, J= 0.4/11 Hz, 1H), 3.70 (dd, *J* = 3.2/11 Hz, 1 H), 4.06 (d, *J =* 16.0 Hz, 1 H), 4.20-4.24 (m, 2H), 4.39-4.51 (m, 2H), 5.32-5.38 (m, 2H), 5.97-6.07 (m, 1H).

### Step 11: Preparation of intermediate 10-hydroxy-5-methyl-3-thia-5,8,10-triazatricyclo[6.2.1.0^{2,6}]undec-2(6)-ene-4,9-dione (1k)

To a solution of 10-allyloxy-5-methyl-3-thia-5,8,10-triaza-tricyclo[6.2.1.0^{2,6}]undec-2(6)-ene-4,9-dione (1j) (0.050 g, 0.19 mmol) and glacial AcOH (0.011 mL, 0.19 mmol) in anhydrous DCM (3.2 mL) was added in one portion Pd(PPh₃)₄ (0.108 g, 0.09 mmol). After stirring for 3 h at rt, the mixture was concentrated and purified by flash chromatography (DCM/acetone 100/0 to 80/20) to provide 10-hydroxy-5-methyl-3-thia-5,8,10-triazatricyclo[6.2.1.0^{2,6}]undec-2(6)-ene-4,9-dione (1k) (0.022 g, 0.10 mmol, 51%).

MS *m*/*z* ([M+H]⁺) 228.

¹H NMR (300 MHz, CDCl₃): *δ* (ppm) 3.13 (s, 3H), 3.24 (d, J= 10.8 Hz, 1H), 3.66 (dd, J= 2.9/10.9 Hz, 1 H), 4.01 (d, J= 16.7 Hz, 1 H), 4.15 (s, 1 H), 4.18 (d, J= 16.8 Hz, 1H).

### Step 12: Preparation of mono-(5-methyl-4,9-dioxo-3-thia-5,8,10-triazatricyclo[6.2.1.0^{2,6}]undec-2(6)-en-10-yl) ester sulfonate sodium salt (Exemple 1)

To a solution of 10-hydroxy-5-methyl-3-thia-5,8,10-triaza-tricyclo[6.2.1.0^{2,6}]undec-2(6)-ene-4,9-dione (1k) (0.022 g, 0.10 mmol) in anhydrous DCM (1.5 mL) was added a suspension of sulfur trioxide pyridine complex (0.092 mg, 0.58 mmol) in dry pyridine (1 mL) and the resulting solution was protected from light and stirred overnight at rt until the sulfatation was completed. The reaction mixture was concentrated under vacuum, diluted with DCM and filtered. The filtrate was co-evaporated with toluene and dried under vacuum. The residue was solubilized in a minimal volume of water and applied on a Dowex sodium form column (Dowex® 50WX8 hydrogen form stored with an aqueous solution of 2N NaOH and washed until neutral pH with water). The fractions containing the desired compound were combined, frozen and lyophilized to afford mono-(5-methyl-4,9-dioxo-3-thia-5,8,10-triaza-tricyclo[6.2.1.0^{2,6}]undec-2(6)-en-10-yl) ester sulfonate sodium salt (Example 1) (0.032 g, 0.10 mmol, quantitative yield) as a white solid.

MS *m*/*z* ([M-H]⁻) 306.

¹H NMR (300 MHz, D₂O): *δ* (ppm) 3.20 (s, 3H), 3.58 (d, *J =* 11.5 Hz, 1 H), 3.84 (dd, *J* = 3.0/11.5 Hz, 1 H), 4.29 (d, *J =* 16.9 Hz, 1 H), 4.40 (d, *J =* 16.9 Hz, 1 H), 4.80 (bs, in D₂O peak, 1H).

### Example 2: biological activity

### Method 1: β-lactamase inhibitory activity - determination of IC₅₀ (table 1)

Enzyme activity was monitored by spectrophotometric measurement of nitrocefin (NCF - TOKU-E, N005) hydrolysis at 485 nm, at room temperature and in assay buffer A: 100mM Phosphate pH7, 2% glycerol and 0.1 mg/mL Bovine serum albumin (Sigma, B4287). Enzymes were cloned in *E. coli* expression vector, expressed and purified in house using classical procedures. To a transparent polystyrene plate (Corning, 3628) were added in each well 5 µL DMSO or inhibitor dilutions in DMSO and 80 µL enzyme in buffer A. Plates were immediately read at 485 nm in a microplate spectrophotometer (BioTek, PowerWave HT) to enable background subtraction. After 30min of pre-incubation at room temperature, 15 µL of NCF (200µM final) were finally added in each well. Final enzyme concentrations were 0.1 nM (TEM-1), 0.4nM (CTX-M-15), 1nM (KPC-2), 0.2nM (P99 AmpC) and 0.3nM (OXA-48). After 20min incubation at room temperature, plates were once again read at 485 nm. Enzyme activity was obtained by subtracting the final signal by the background, and was converted to enzyme inhibition using non inhibited wells. IC₅₀ curves were fitted to a classical Langmuir equilibrium model with Hill slope using XLFIT (IDBS).

**Table 1: IC₅₀ (nM) for β-lactamase Inhibitory Activity**

| Compound example | IC₅₀ (nM) | | | | |
|---|---|---|---|---|---|
| | TEM-1 | CTX-M-15 | KPC-2 | P99 AmpC | OXA-48 |
| 1 | 3.3 | 0.69 | 3.3 | 48 | 0.89 |

### Method 2: Synergy with ceftazidime against bacterial isolates (table 2 and table 3)

Compounds of the present invention were assessed against genotyped bacterial strains in combination with the β-lactam ceftazidime. In the assays, MICs of ceftazidime at fixed concentrations of said compounds were determined by the broth microdilution method according to the Clinical Laboratory Standards Institute (CLSI - M7-A7). Briefly, compounds and ceftazidime dilutions were prepared in DMSO and spotted (1µL each) on sterile polystyrene plates (Corning, 3788). Log phase bacterial suspensions were adjusted to a final density of 5x10⁵ cfu/mL in cation-adjusted Mueller-Hinton broth (Beckton-Dickinson) and added to each well (98µL). Microplates were incubated for 16-20 h at 35°C in ambient air. The MIC of ceftazidime at each compound concentration was defined as the lowest concentration of ceftazidime that prevented bacterial growth as read by visual inspection.

**Table 2: Bacterial isolates**

| ID | Organism | Enzyme |
|---|---|---|
| 1 | *E. coli* | TEM-1 / SHV-12 / CTX-M-15 / OXA-1 |
| 2 | *E. cloacae* | P99 AmpC |

**Table 3: Synergy MIC assay**

| | MIC (µg/mL) | |
|---|---|---|
| | *E. coli* TEM-1 / SHV-12 / CTX-M-15 / OXA-1 | *E. cloacae* P99 AmpC |
| Ceftazidime | 64 | 256 |
| Ceftazidime + example 1 compound | (4µg/mL) <=0.125 | 0.5 |

## Claims

1. A compound of formula (I) 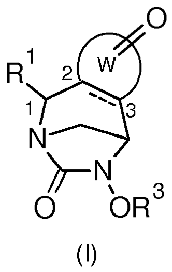 wherein
• W, unsubstituted or substituted by one or more T, represents a non-aromatic, unsaturated 5- or 6-membered heterocycle comprising at least one group N-R² and a group (X)ₙ ;
• X, identical or different, independently represents C(O), O, N, N(R²), S, S(O) or S(O)₂ ;
• R¹ represents a carbon-linked, unsubstituted or substituted by one or more T¹, aromatic, saturated, totally or partially unsaturated 4-, 5- or 6-membered heterocycle comprising at least one nitrogen atom ; a hydrogen atom ; -CN ; - C(O)NHQ¹ ; -C(O)NHOQ¹ ; -C(O)NH-NHQ¹ ; -C(O)O-NHQ¹ ; -C(O)OQ¹ ; - (CH₂)ₘOC(O)OQ¹ ; -(CH₂)ₘOQ¹ ; -(CH₂)ₘOC(O)Q¹ ; -(CH₂)ₘOC(O)NQ¹Q² ; - (CH₂)ₘ-NHC(O)Q¹ ; -(CH₂)ₘNHS(O)₂Q¹ ; -(CH₂)ₘNHS(O)₂NQ¹Q² ; - (CH₂)ₘNHC(O)OQ¹ ; -(CH₂)ₘNHC(O)NQ¹Q²; -(CH₂)ₘNHQ³ ; -(CH₂)ₘNH-C(NHQ³)=NQ⁴ ; -(CH₂)ₘNH-CH=NQ³ ; -C(NHQ³)=NQ⁴ ;
• R², identical or different, independently represents -(CH₂)_{q}OQ⁵ ; -(CH₂)ᵥ-CN ; - (CH₂)_{q}OC(O)Q⁵ -(CH₂)ᵥ-C(O)OQ⁵ ; -(CH₂)_{q}-OC(O)OQ⁵; -(CH₂)_{q}-OC(O)NQ⁵Q⁶; - (CH₂)ᵥ-C(O)NQ⁵Q⁶; -(CH₂)ᵥ-C(O)ONQ⁵; -(CH₂)ᵥ-C(O)NHOQ⁵; -(CH₂)ᵥ-C(O)NH-NHQ⁵ ; -(CH₂)ᵥ-C(O)O-NHQ⁵; -(CH₂)_{q}-NHC(O)Q⁵ , -(CH₂)_{q}NHS(O)₂Q⁵; - (CH₂)_{q}NHS(O)₂NQ⁵Q⁶ -(CH₂)_{q}-NHC(O)OQ⁵ ; -(CH₂)_{q}-NHC(O)NQ⁵Q⁶ ; - (CH₂)_{q}NQ⁵Q⁶; -(CH₂)_{q}-NH-C(NHQ³)=NQ⁴ ; -(CH₂)_{q}-NH-CH=NQ³ ; (CH₂)ᵥ-C(NHQ³)=NQ⁴ ; or
R², identical or different, independently unsubstituted or substituted by one or more T², independently represents C₁-C₃-alkyl ; C₁-C₃-fluoroalkyl ; -(CH₂)ₚ-C₃-C₆-cycloalkyl ; -(CH₂)ₚ-C₃-C₆-cyclofluoroalkyl ; -(CH₂)ₚ-(4-, 5- or 6-membered aromatic, saturated, totally or partially unsaturated heterocycle) ;
• R³ represents SO₃H, CFHCO₂H or CF₂CO₂H ;
• Q¹ and Q², identical or different, independently represent a hydrogen atom ; - (CH₂)qNHQ³ ; -(CH₂)_{q}-NH-C(NHQ³)=NQ⁴ ; (CH₂)_{q}-NH-CH=NQ³ ; (CH₂)ᵥ-C(NHQ³)=NQ⁴ ; -(CH₂)_{q}OQ³ ; -(CH₂)ᵥCONHQ³ ; or
Q¹ and Q², identical or different, independently unsubstituted or substituted by one or more T², independently represent a C₁-C₃-alkyl ; -(CH₂)ₚ-(4-, 5- or 6-membered heterocycle) ; or
Q¹, Q² and the nitrogen atom to which they are bonded, form a saturated or partially unsaturated 4-, 5- or 6-membered heterocycle comprising 1, 2, 3 or 4 heteroatoms ;
• Q³ and Q⁴, identical or different, independently represent a hydrogen atom or a C₁-C₃-alkyl ;
• Q⁵ and Q⁶, identical or different, independently represent a hydrogen atom ; - (CH₂)_{q}NHQ³ ; -(CH₂)_{q}-NH-C(NHQ³)=NQ⁴ ; (CH₂)_{q}-NH-CH=NQ³ ; (CH₂)ᵥ-C(NHQ³)=NQ⁴ ; -(CH₂)_{q}OQ³ ; -(CH₂)ᵥCONHQ³ ; or
Q⁵ and Q⁶, identical or different, independently unsubstituted or substituted by one or more T², independently represent a C₁-C₃-alkyl ; -(CH₂)ₚ-(4-, 5- or 6-membered heterocycle) ; or
Q⁵, Q⁶ and the nitrogen atom to which they are bonded, form a saturated or partially unsaturated 4-, 5- or 6-membered heterocycle comprising 1, 2, 3 or 4 heteroatoms ;
• T¹, identical or different, independently represents a fluorine atom ; -(CH₂)ₚOQ¹ ; - (CH₂)ₚ-CN ; -(CH₂)ₚOC(O)Q¹ ; -(CH₂)ₚ-C(O)OQ¹ ; -(CH₂)ₚ-OC(O)OQ¹ ; -(CH₂)ₚ-OC(O)NHQ¹; -(CH₂)ₚ-C(O)NHQ¹ ; -(CH₂)ₚ-C(O)NHOQ¹ ; -(CH₂)ₚ-C(O)NH-NHQ¹ ; - (CH₂)ₚ-C(O)O-NHQ¹, ; -(CH₂)ₚ-NHC(O)Q¹ ; -(CH₂)ₚNHS(O)₂Q¹ ; - (CH₂)ₚNHS(O)₂NQ¹Q²; -(CH₂)ₚ-NHC(O)OQ¹ ; -(CH₂)ₚ-NHC(O)NQ¹Q²; - (CH₂)ₚNHQ¹ ; -(CH₂)ₚ-NH-C(NHQ³)=NQ⁴ ; -(CH₂)ₚ-NH-CH=NQ³; (CH₂)ₚ-C(NHQ³)=NQ⁴ ; or
T¹, unsubstituted or substituted by one or more T², identical or different, independently represents C₁-C₃-alkyl; C₁-C₃-fluoroalkyl ; O-C₁-C₃-fluoroalkyl ; - (CH₂)ₚ-(4-, 5- or 6-membered aromatic, saturated, totally or partially unsaturated heterocycle comprising at least one nitrogen atom) ;
• T², identical or different, independently represents OH ; NH₂ or CONH₂ ;
• T, identical or different, independently represents a fluorine atom ; C₁-C₃-alkyl ; C₁-C₃-fluoroalkyl ; O-C₁-C₃-fluoroalkyl ; -(L)_{w}-(CH₂)ₚ-C₃-C₆-cycloalkyl ; -(L)_{w}-(CH₂)ₚ-C₃-C₆-cyclofluoroalkyl ; -(L)_{w}-(CH₂)ₚ-heterocycle ; -(L)_{w}-(CH₂)ₚ-CN ; -(L)_{w}-(CH₂)ₚOC(O)Q⁵; -(L)_{w}-(CH₂)ₚ-C(O)OQ⁵ ; (L)_{w}-(CH₂)ₚ-OC(O)OQ⁵; -(L)_{w}(CH₂)ₚ-OC(O)NQ⁵Q⁶; -(L)_{w}-(CH₂)ₚ-C(O)NQ⁵Q⁶ ; -(L)_{w}-(CH₂)ₚ-C(O)NQ⁵OQ⁶ ; -(L)_{w}-(CH₂)ₚ-C(O)NQ⁵-NQ⁵Q⁶ ; -(L)_{w}-(CH₂)ₚ-NQ⁵C(O)Q⁶ ; -(L)_{w}-(CH₂)ₚNQ⁵Q(O)₂Q⁶; -(L)_{w}-(CH₂)ₚ-NQ⁵C(O)OQ⁶; -(L)_{w}-(CH₂)ₚ-NQ⁵C(O)NQ⁵Q⁶ ; -(L)_{w}-(CH₂)ₚNQ⁵Q⁶; -(L)_{w}-(CH₂)ₚ-NH-C(NHQ³)=NQ⁴ ; -(L)_{w}-(CH₂)ₚ-NH-CH=NQ³ ; -(L)_{w}(CH₂)ₚ-C(NHQ³)=NQ⁴;
• L, identical or different, independently represents O, S, N(R²), S(O) or S(O)₂ ;
• m represents 1 or 2 ;
• n represents 0, 1 or 2 ;
• p, identical or different, independently represents 0, 1, 2 or 3 ;
• q, identical or different, independently represents 2 or 3 ;
• v, identical or different, independently represents 1, 2 or 3 ;
• w, identical or different, independently represents 0 or 1 ;
wherein
▪ any carbon atom present within a group selected from alkyl, cycloalkyl, fluoroalkyl, cyclofluoroalkyl and heterocycle can be oxidized to form a C=O group ;
▪ any sulphur atom present within a heterocycle can be oxidized to form a S=O group or a S(O)₂ group ;
▪ any nitrogen atom present within a heterocycle or present within group wherein it is trisubstituted thus forming a tertiary amino group, can be further quaternized by a methyl group ;
and a pharmaceutically acceptable salt, a zwitterion, an optical isomer, a racemate, a diastereoisomer, an enantiomer, a geometric isomer or a tautomer thereof.

2. A compound according to claim 1 selected from the compounds of formulae (A), (B), (I*), (A*) and (B*) 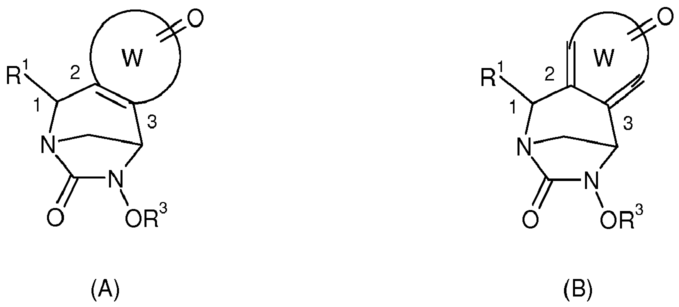 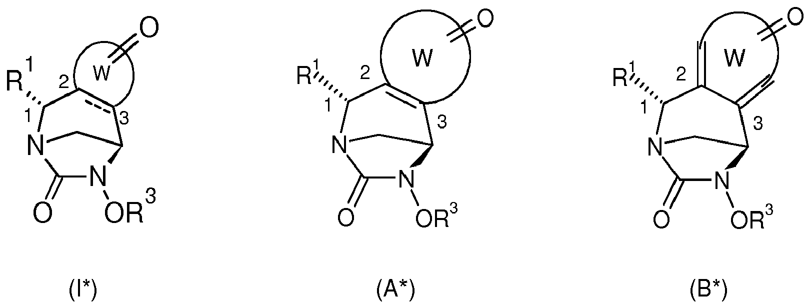 wherein W, unsubstituted or substituted by one or more T, X, n, R¹, R² and R³ are defined according to claim 1.

3. A compound according to one of claims 1 and 2 that is selected from
• a compound of formula (I) wherein W, unsubstituted or substituted by one or more T, represents a non-aromatic, unsaturated 5-membered heterocycle comprising a group N-R² and a group (X)ₙ ;
• a compound of formula (I) wherein W, unsubstituted or substituted by one or more T, represents a non-aromatic, unsaturated 6-membered heterocycle comprising a group N-R² and a group (X)ₙ ;
• a compound of formula (A) wherein W, unsubstituted or substituted by one or more T, represents a non-aromatic, unsaturated 5-membered heterocycle comprising a group N-R² and a group (X)ₙ ;
• a compound of formula (A) wherein W, unsubstituted or substituted by one or more T, represents a non-aromatic, unsaturated 6-membered heterocycle comprising a group N-R² and a group (X)ₙ ;
• a compound of formula (B) wherein W, unsubstituted or substituted by one or more T, represents a non-aromatic, unsaturated 6-membered heterocycle comprising a group N-R² and a group (X)ₙ ;
• a compound of formula (I*) wherein W, unsubstituted or substituted by one or more T, represents a non-aromatic, unsaturated 5-membered heterocycle comprising a group N-R² and a group (X)ₙ ;
• a compound of formula (I*) wherein W, unsubstituted or substituted by one or more T, represents a non-aromatic, unsaturated 6-membered heterocycle comprising a group N-R² and a group (X)ₙ ;
• a compound of formula (A*) wherein W, unsubstituted or substituted by one or more T, represents a non-aromatic, unsaturated 5-membered heterocycle comprising a group N-R² and a group (X)ₙ ;
• a compound of formula (A*) wherein W, unsubstituted or substituted by one or more T, represents a non-aromatic, unsaturated 6-membered heterocycle comprising a group N-R² and a group (X)ₙ ;
a compound of formula (B*) wherein W, unsubstituted or substituted by one or more T, represents a non-aromatic, unsaturated 6-membered heterocycle comprising a group N-R² and a group (X)ₙ ;
and wherein W, unsubstituted or substituted by one or more T, X, R¹, R², R³ and T are defined according to claim 1.

4. A compound according to one of claims 1 to 3 selected from the compounds of formulae (A1) to (A68) and (B1) to (B8) 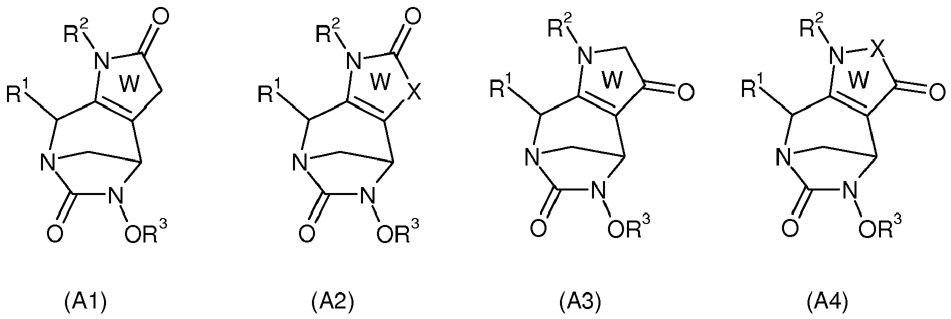 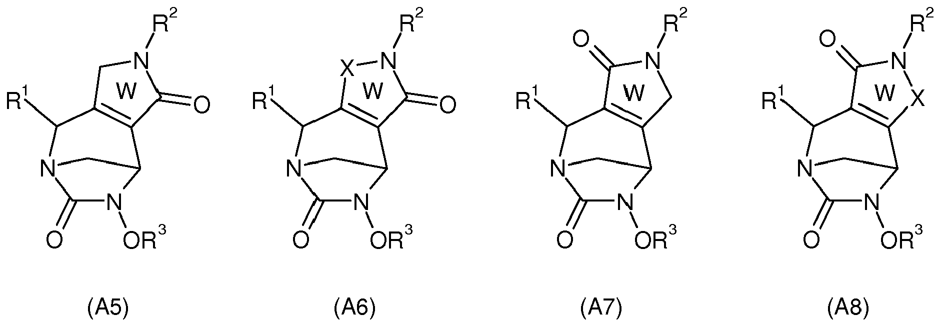 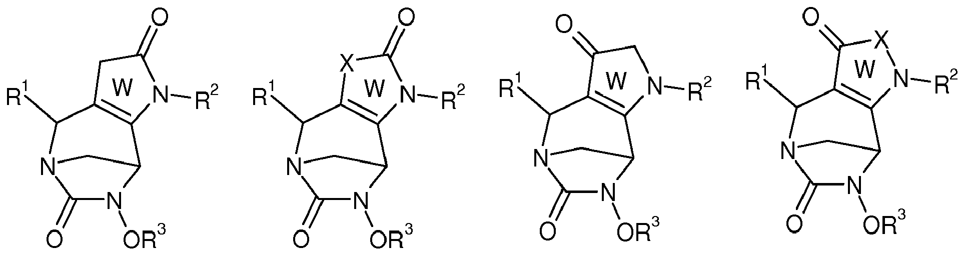 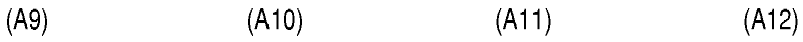 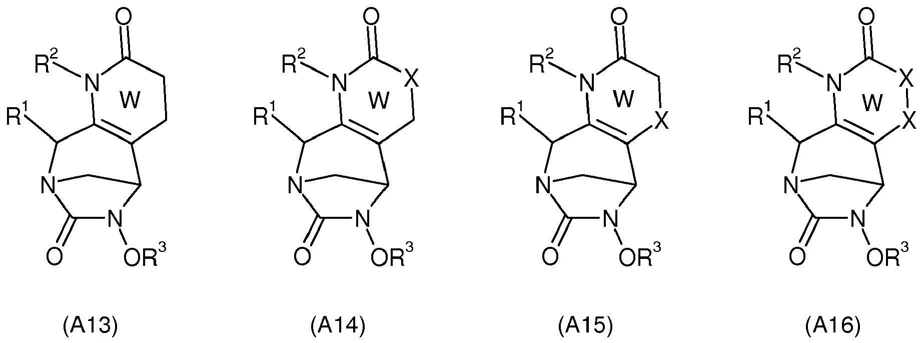 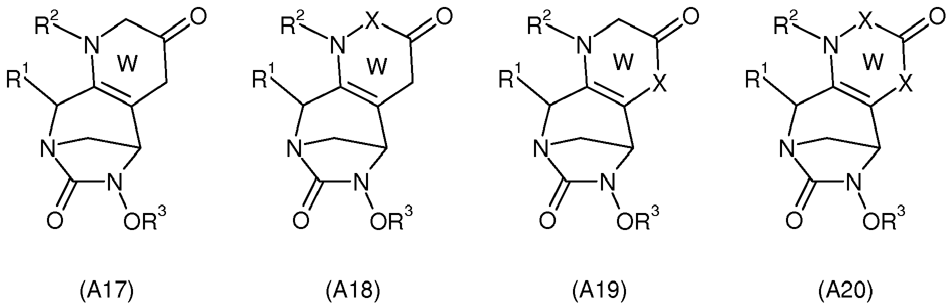 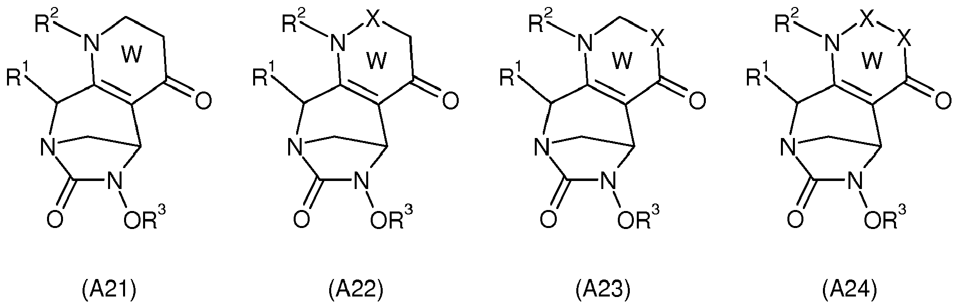 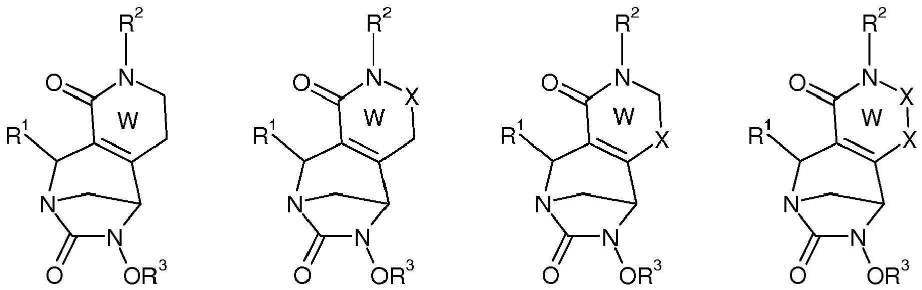 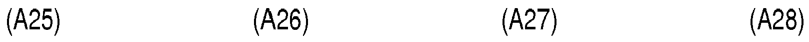 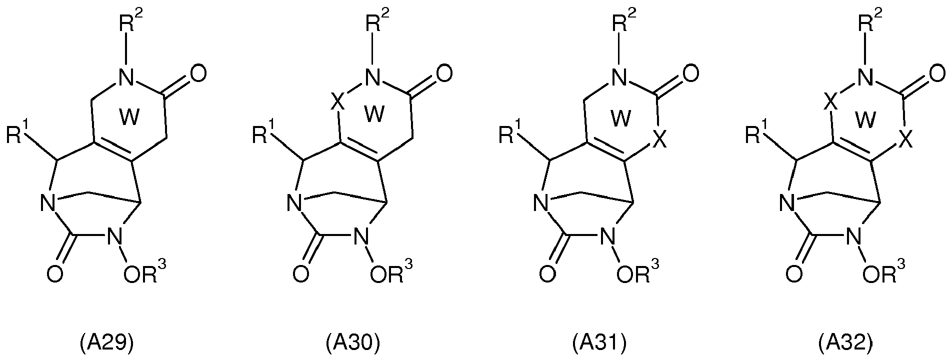 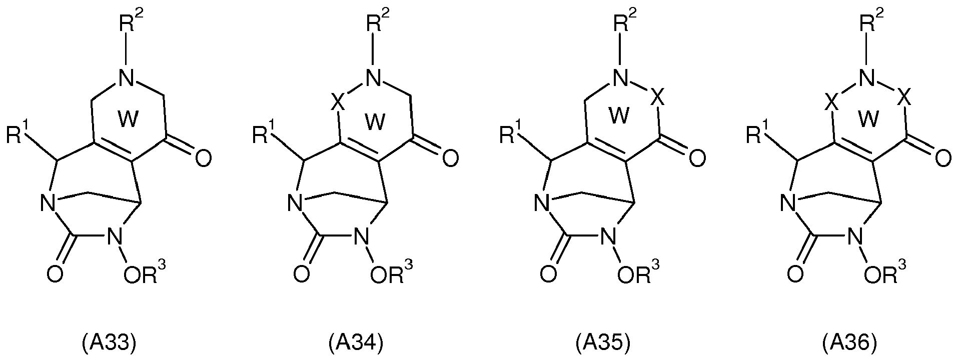 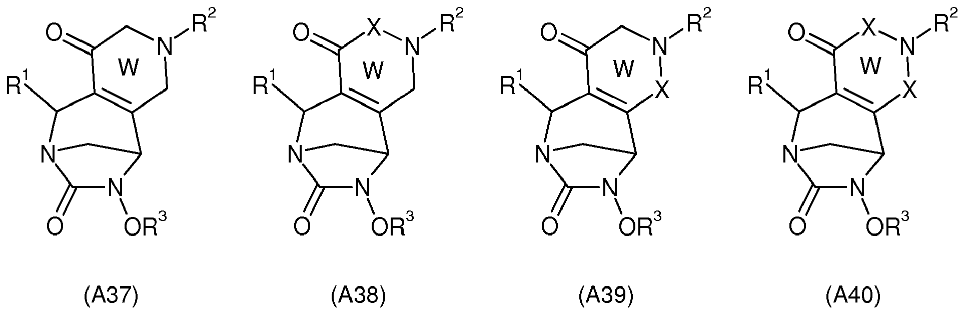 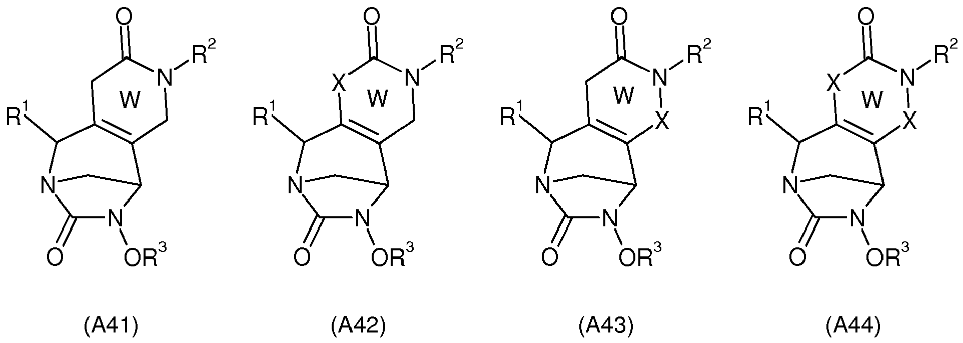 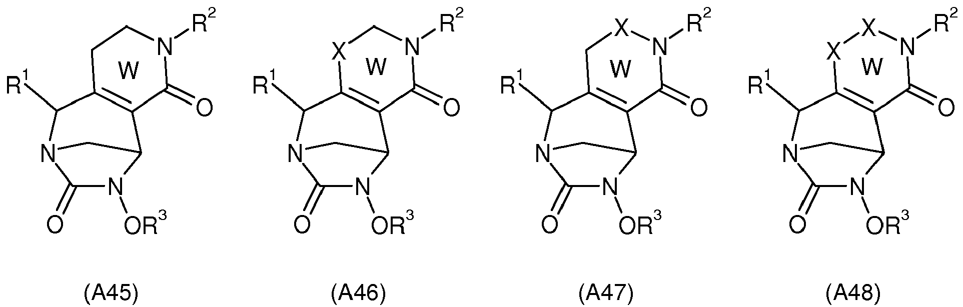 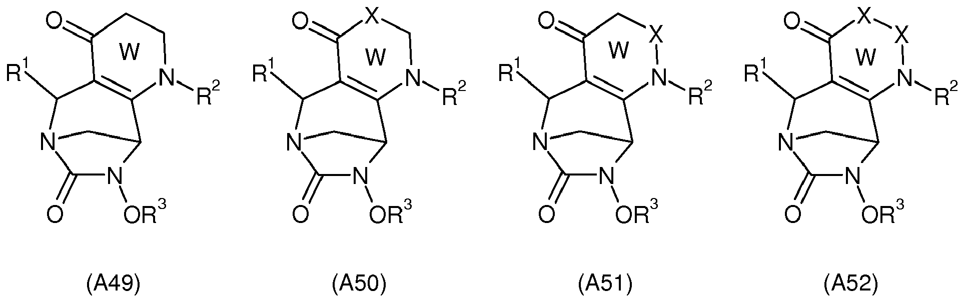 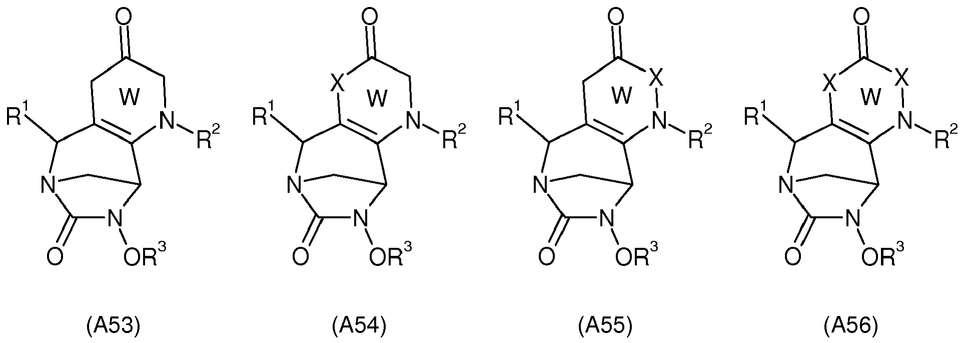 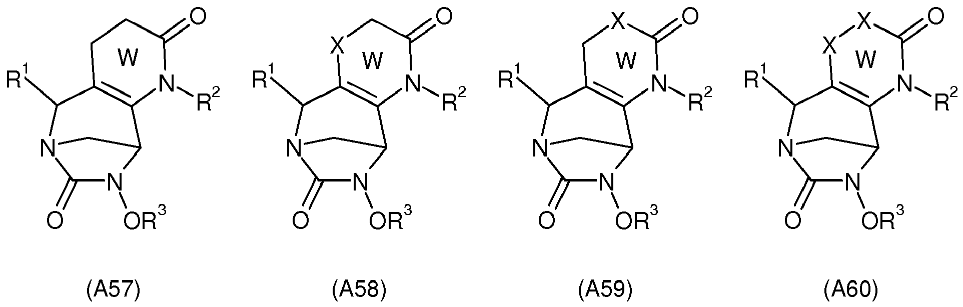 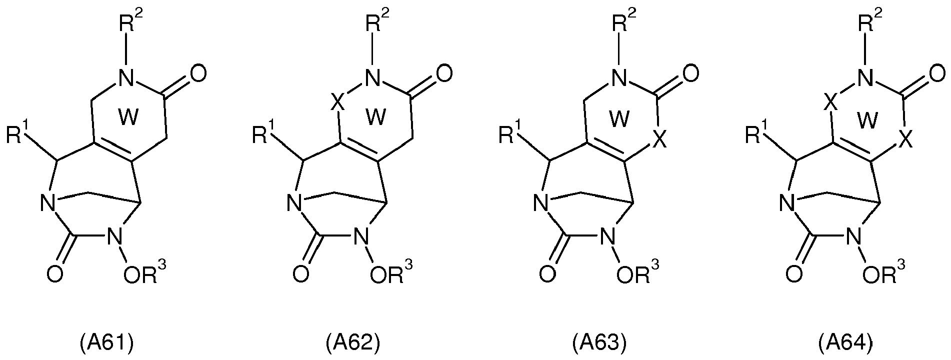 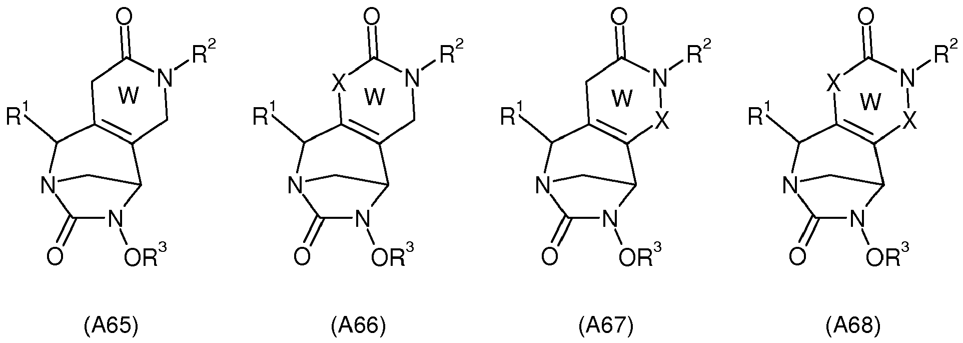 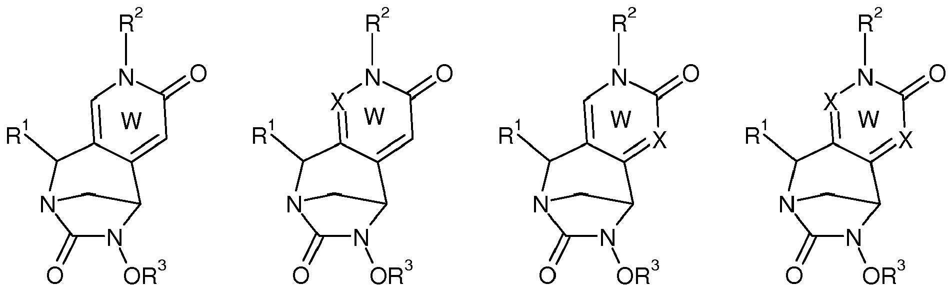 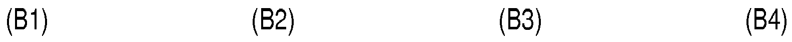 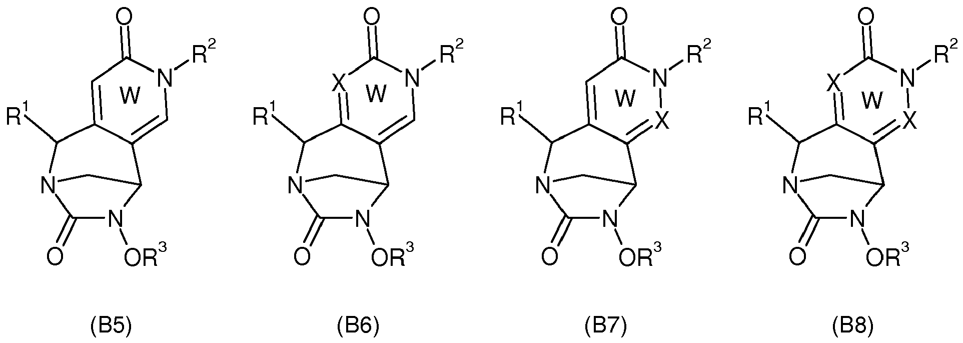 wherein W, unsubstituted or substituted by one or more T, X, R¹, R², R³ and T are defined according to claim 1.

5. A compound according to one of claims 1 to 4 wherein
▪ R¹ represents a carbon-linked, unsubstituted or substituted by one or more T¹, aromatic, saturated, totally or partially unsaturated 4-, 5- or 6-membered heterocycle comprising at least one nitrogen atom ; a hydrogen atom ; -CN ; - C(O)NHQ¹ ; -C(O)NHOQ¹ ; -C(O)NH-NHQ¹ ; -C(O)O-NHQ¹ ; -C(O)OQ¹ ; - (CH₂)OC(O)OQ¹ ; -(CH₂)₂OC(O)OQ¹ ; -(CH₂)OQ¹ ; -(CH₂)₂OQ¹ ; -(CH₂)OC(O)Q¹ ; - (CH₂)₂OC(O)Q¹ ; -(CH₂)-OC(O)NQ¹Q² ; -(CH₂)₂OC(O)NQ¹Q² ; -(CH₂)NHC(O)Q¹ ; - (CH₂)₂-NHC(O)Q¹ ; -(CH₂)NHS(O)₂Q¹ ; -(CH₂)₂NHS(O)₂Q¹ ; - (CH₂)NHS(O)₂NQ¹Q² ; -(CH₂)₂NHS(O)₂Q¹Q² ; -(CH₂)NHC(O)OQ¹ ; - (CH₂)₂NHC(O)OQ¹ ; -(CH₂)NHC(O)NQ¹Q²; -(CH₂)₂NHC(O)NQ¹Q² ; preferably a carbon-linked, unsubstituted or substituted by one or more T¹, aromatic, saturated, totally or partially unsaturated 4-, 5- or 6-membered heterocycle comprising at least one nitrogen atom ; -CN ; -C(O)NHQ¹ ; -C(O)NHOQ¹ ; -C(O)NH-NHQ¹ ; - (CH₂)₂OQ¹ ; or
▪ R¹ represents -(CH₂)NHQ³ ; -(CH₂)₂NHQ³ ; -(CH₂)NH-C(NHQ³)=NQ⁴ ; -(CH₂)₂NH-C(NHQ³)=NQ⁴ ; -(CH₂)NH-CH=NQ³ ; -(CH₂)₂NH-CH=NQ³ ; -C(NHQ³)=NQ⁴, preferably represents -(CH₂)NHQ³ ; -(CH₂)NH-C(NHQ³)=NQ⁴.

6. A pharmaceutical composition comprising at least one compound according to one of claims 1 to 5.

7. A pharmaceutical composition according to claim 6 further comprising at least one compound selected from an antibacterial compound, preferably a β-lactam compound.

8. A pharmaceutical composition according to one of claims 6 and 7 comprising
• a single compound according to one of claims 1 to 5 ;
• a compound according to one of claims 1 to 5 and one or more antibacterial compound ;
• a compound according to one of claims 1 to 5 and one or more β-lactam compound ;
• a compound according to one of claims 1 to 5, one or more antibacterial compound and one or more β-lactam compound.

9. A pharmaceutical composition according to one claims 7 and 8 wherein
• the antibacterial compound is selected from aminoglycosides, β-lactams, glycylcyclines, tetracyclines, quinolones, fluoroquinolones, glycopeptides, lipopeptides, macrolides, ketolides, lincosamides, streptogramins, oxazolidinones, polymyxins and mixtures thereof; or
• the β-lactam compound is selected from β-lactams and mixtures thereof, preferably penicillin, cephalosporins, penems, carbapenems and monobactam.

10. A pharmaceutical composition according to claim 6 further comprising at least one compound according to one of claims 1 to 5 and a pharmaceutically acceptable excipient.

11. A kit comprising a pharmaceutical composition according to one of claims 6 to 10 and at least one second composition according to one of claims 6 to 10.

12. A compound or a composition according to one of claims 1 to 10 for its use as a medicine or for its use for treating or preventing a bacterial infection.

13. A compound or a composition according to claim 12 for its use for treating or preventing a bacterial infection caused by bacteria producing one or more β-lactamase.

14. A compound or a composition according to one of claims 12 and 13 for its use for treating or preventing a bacterial infection caused by a gram-positive bacteria or by gram-negative bacteria, preferably a bacterial infection caused by gram-negative bacteria.

15. A kit according to claim 11 for the treatment or prevention of bacterial infections by its simultaneous, separate or sequential administration to a patient in need thereof.
